# EUROPEAN PATENT APPLICATION

(11) **EP 3 381 907 A1**
(43) Date of publication of application: **03.10.2018**
(21) Application number: 17163014.8
(22) Date of filing: 27.03.2017
(51) Int. Cl.: C07D 235/18, C07D 263/57, C07F 5/02, A61K 31/423, A61P 35/00

(54) **SYMMETRICAL 2-AMINOPHENYL-BENZAZOLYL-5-ACETATE COMPOUNDS AND THEIR USE AS ANTI-HEPARANASE**

(71) Applicant: Leadiant Biosciences SA, 6850 Mendrisio (CH)
(72) Inventor: GIANNINI, Giuseppe, 00071 Pomezia (ROMA) (IT); BATTISTUZZI, Gianfranco, 00121 Ostia (ROMA) (IT); DI SANTO, Roberto, 00121 Ostia (ROMA) (IT); COSTI, Roberta, 00121 Ostia (ROMA) (IT); MESSORE, Antonella, 03040 Vallemaio (FR) (IT)
(74) Representative: Ghirardi, Valeria

(57) **Abstract**

The present invention relates to symmetrical 2-aminophenyl-benzazolyl-5-acetate compounds of formula (I).

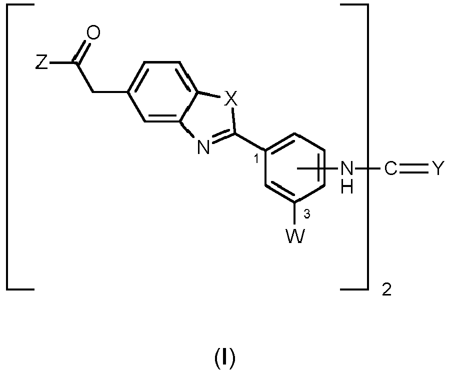

Such compounds are endowed with an anti-heparanase activity. The present invention also relates to the use of such compounds as a medicament, in particular for the treatment of diseases and disorders associated with a heparanase activity, and to pharmaceutical compositions comprising the same.

## Description

### FIELD OF THE INVENTION

The present invention relates to symmetrical 2-aminophenyl-benzazolyl-5-acetate compounds having an anti-heparanase activity.

The invention also relates to the use of such compounds as a medicament, in particular for the treatment of diseases and disorders associated with heparanase activity, and to pharmaceutical compositions comprising the same.

### BACKGROUND OF THE INVENTION

Heparan sulfate proteoglycans (HSPGs) are major components of the basement membrane and extracellular matrix. They are ubiquitous macromolecules associated with the cell surface and extracellular matrix (ECM) of a wide range of cells of vertebrate and invertebrate tissues (Bernfield et al. Annu. Rev. Biochem. 1999, 68, 729; lozzo R.V., San Antonio J.D. J. Clin. Invest. 2001, 108, 349; Kjellen L., Lindahl U. Annu. Rev. Biochem. 1991, 60, 443). HSPGs are a class of carbohydrate-modified proteins involved in key biological processes, including a role as co-receptors for a number of ligand molecules to regulate their signaling and distribution (Nakato H., Li J.P. Int. Rev. Cell. Mol. Biol. 2016, 325, 275).

Heparan sulfate (HS) is a component of cell surface and matrix-associated proteoglycans (HSPGs). A key function of HS is to bind and interact with signaling proteins, growth factors, plasma proteins, immune-modulators and other factors. In doing so, the HS chains and HSPGs are able to regulate protein distribution, bio-availability and action on target cells. Moreover, the HS chains ensure that a wide variety of bioactive molecules bind to the cell surface and ECM and thereby function in the control of normal and pathological processes, among which are morphogenesis, tissue repair, inflammation, vascularization and cancer metastasis (Billings P.C., Pacifici M. *Connect Tissue Res.* 2015, *56(4)*, 272). HS glycosaminoglycans bind to and assemble a multitude of ECM proteins (i.e., laminin, fibronectin, collagen type IV) and thereby significantly contribute to the ECM self assembly and integrity.

Heparanase is an endoglycosidase which cleaves heparan sulfate (HS) and hence participates in degradation and remodeling of the extracellular matrix (ECM), with a release of bioactive saccharide fragments and HS-bound growth factors and cytokines. Heparanase is preferentially expressed in human tumors and its over-expression in tumor cells confers an invasive phenotype in experimental animals. Heparanase upregulation correlates with increased tumor vascularity and poor postoperative survival of cancer patients. Heparanase is synthesized as a 65 kDa inactive precursor that undergoes proteolytic cleavage, yielding 8 kDa and 50 kDa protein subunits that heterodimerize to form an active enzyme.

Heparanase exhibits also non-enzymatic activities, independent of its involvement in ECM degradation.

There is growing evidence that heparanase upregulates expression of genes that participate in cancer metastasis and angiogenesis, glucose metabolism, immune response, inflammation and atherosclerosis, suggesting that heparanase belongs to an emerging class of proteins that play a significant role in regulating transcription in addition to their well-recognized extra-nuclear functions (Pisano C. et al. Biochem. Pharmacol. 2014, 89, 12; Ilan N. et al. Int. J. Biochem. Cell Biol. 2006, 38, 2018; Fux L. et al. Trends Biochem. Sci. 2009, 34, 511; Parish C.R. et al. Matrix Biol. 2013, 32, 228; Ramani V.C. et al. FEBS J. 2013, 280, 2294; Vlodavsky I. et al. Matrix Biol. 2013. 32, 241; Yang Y. et al. Cancer Res. 2010, 70, 8329).

In recent years, heparanase has attracted considerable attention as a promising target for innovative pharmacological applications. Indeed, heparanase appears to be involved in major human diseases, from tumors to chronic inflammation, diabetic nephropathy, bone osteolysis, thrombosis and atherosclerosis, in addition to more recent investigation in various rare diseases (Rivara S., Milazzo F.M., Giannini G. Future Med Chem. 2016, 8(6), 647. "Heparanase: a rainbow pharmacological target associated to multiple pathologies including rare diseases").

In view of the above, compounds able to specifically modulate the activity of this enzyme are highly desired as a useful pharmacological option for many therapeutic indications. Since heparanase is involved in a very wide range of molecular pathways, modulation of the expression or activity of this enzyme is a highly viable therapeutic option.

Potent and selective heparanase inhibitors are therefore highly searched as therapeutic tools for those clinical indications in which heparanase inhibition is pharmacologically useful.

However, although in the last twenty years numerous efforts have been made in this sense and huge developments have been made in the knowledge of heparanase activity and functions, so far no drug able to inhibit or modulate heparanase functions has yet been registered (Rivara S. et al, Future Med Chem. 2016, 8(6), 647).

Some compounds are known in the art as heparanase inhibitors.

In particular, three classes of heparanase inhibitors are known: active analogous of endogenous substance, synthetic small molecule compounds and natural products. In the literature, monoclonal antibodies and nucleic acid-based inhibitors of heparanase are also mentioned.

Among these known compounds, only a few heparin derivatives have so far reached the stage of clinical trial (see for example the following reviews: Ferro V. Mini Rev. Med. Chem. 2004, 4, 693; Jia L., Ma S. Eur. J. Med. Chem. 2016, 121, 209; Rivara S. et al. Future Med. Chem. 2016, 8, 647).

However, the heparin-derivatives macromolecules currently under clinical investigation have a high molecular weight.

Small molecules, instead, are particularly desirable due to their more favorable pharmacokinetic properties. Furthermore, they can be administered orally thus resulting in an improved patient compliance.

Indeed, progress in the development of drugs able to inhibit heparanase activity has been limited also by the lack of efficient small molecule inhibitors.

Therefore, there is still the need of small molecules able to inhibit heparanase activity.

Among synthetic small molecules compounds, some urea derivatives have been designed and their heparanase inhibition activity has been studied.

For example, Pan's group (Pan W., Miao H.Q., Xu Y.J., Navarro E.C., Tonra J.R., Corcoran E., Lahiji A., Kussie P., Kiselyov A.S., Wong W.C., Liu H. Bioorg. Med. Chem. Lett. 2006, 16(2), 409), described a class of 1-[4-(1H-benzoimidazol-2-yl)-phenyl]-3-[4-(1H-benzoimidazol-2-yl)-phenyl]-ureas and investigated heparanase inhibitory activity of some compounds of this class. In Xu Y.J. et al. Bioorg. Med. Chem. Lett. 2006, 16(2), 404, a class of N-(4-{[4-(1H-benzoimidazol-2-yl)-arylamino]-methyl}-phenyl)-benzamides is described and some compounds are tested as heparanase inhibitors.

In "3D QSAR based design of novel substituted urea molecules as heparanase inhibitors" (Bathini R., Fatima S., Sivan S.K., Manga V. J. Pharm. Res. 2013, 7(8), 754) symmetric urea derivatives are designed and their activity on heparanase inhibition is predicted.

In 2005 Courtney's group (Courtney S.M., Hay P.A., Buck R.T., Colville C.S., Phillips D.J., Scopes D.I., Pollard F.C., Page M.J., Bennett J.M., Hircock M.L., McKenzie E.A., Bhaman M., Felix R., Stubberfield C.R., Turner P.R. Bioorg. Med. Chem. Lett. 2005, 15(9), 2295), synthetized and studied a series of furanyl-1,3-thiazol-2-yl and benzoxazol-5-yl acetic acids for their anti-heparanase activity.

In 2004, Courtney S.M. et al. (Courtney S.M., Hay P.A., Buck R.T., Colville C.S., Porter D.W., Scopes D.I., Pollard F.C., Page M.J., Bennett J.M., Hircock M.L., McKenzie E.A., Stubberfield C.R., Turner P.R. Bioorg. Med. Chem. Lett. 2004, 14(12), 3269) described a class of 2,3-dihydro-1,3-dioxo-1H-isoindole-5-carboxylic acids as heparanase inhibitors.

Further derivatives have been disclosed as heparanase inhibitors, for example in the following patent literature.

WO02060374 discloses benzimidazole, benzoxazole and benzothiazole derivatives and their uses as heparanase inhibitors. Also WO2004046123 and WO2004046122 disclose benzimidazole, benzoxazole and benzothiazole derivatives as heparanase inhibitors.

WO2005042495 discloses (benzimidazol-2-yl)-phenyl-phenyl-urea compounds and their uses as heparanase inhibitors.

All the above mentioned documents show that a big effort has been put in the design of small molecules compounds able to efficiently inhibit heparanase and also endowed with good pharmacokinetic properties. It is therefore evident that such molecules are still highly desired.

### SUMMARY OF THE INVENTION

It has now been found that symmetrical 2-aminophenyl-benzazolyl-5-acetate derivatives are able to efficiently inhibit heparanase activity.

It is an object of the present invention a compound having the following formula (I) 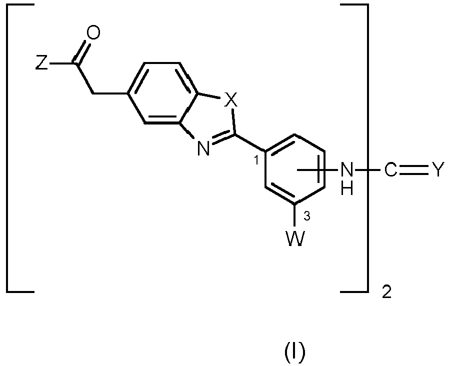
wherein Y is selected from the group consisting of O, S and NH,
X is selected from the group consisting of O and NH,
W is selected from the group consisting of H and F,
Z is selected from the group consisting of OH, NH-CHR-COOH, wherein R is the residue of an amino acid, and NHCHR₂R₃, wherein R₂ is selected from H and CH₂CH(CH₃)₂ and R₃ is selected from H and B(OH)₂,
the enantiomers, diastereoisomers, and mixtures thereof,
or a pharmaceutically acceptable salt, hydrate or solvate thereof.

Indeed, it has been found that such compounds are able to efficiently inhibit heparanase activity.

Preferably, W is fluorine (F).

Preferably, Z is selected from OH and NH-CHR-COOH.

When Z is NH-CHR-COOH, R is preferably a residue of an amino acid selected from glycine, alanine and phenylalanine.

In a preferred embodiment of the invention, the compounds of formula (I) are those in which W is fluorine and Z is selected from the group consisting of OH, NH-CHR-COOH, wherein R is the residue of an amino acid, and NHCHR₂R₃, in particular wherein R₂ is selected from H and CH₂CH(CH₃)₂ and R₃ is B(OH)₂.

In another preferred embodiment of the invention, the compounds of formula (I) are those in which W is F, Y is O, X is O and Z has the meanings above defined. In this embodiment, Z is preferably selected from OH and NH-CHR-COOH, wherein R is the residue of an amino acid preferably selected from glycine, alanine and phenylalanine.

In another preferred embodiment, the compounds of formula (I) are those in which W is F, Y is O, X is NH and Z has the meanings above defined. In this embodiment, Z is preferably selected from OH and NH-CHR-COOH, wherein R is a residue of an amino acid, said amino acid being preferably phenylalanine.

In another preferred embodiment, the compounds of formula (I) are those in which W is F, Y is S, X is O and Z has the meanings above defined. In this embodiment, Z is preferably NH-CHR-COOH, wherein R is a residue of an amino acid, said amino acid being preferably glycine.

In another preferred embodiment, the compounds of formula (I) are those in which W is F, Y is S, X is NH and Z has the meanings above defined. In this embodiment, Z is preferably selected from OH and NH-CHR-COOH wherein Rhas the meanings above defined.

A process for the preparation of the compound of formula (I), as will be better defined below, is also an object of the present invention.

It is also an object of the invention the compound of formula (I) for use as a medicament.

A further object of the present invention is the compound of formula (I) for use for the treatment of a disease which can be improved or prevented by an anti-heparanase activity.

In a preferred embodiment, said disease is selected from the group consisting of tumor, primary tumor, metastases, diabetic retinopathy, psoriasis, retrolenticular fibroplasia, restenosis after angioplasty, coronary by-pass, inflammation, sepsis, arthritis, autoimmune diseases, allograft rejection, cardiovascular diseases, fibro-proliferative disease, diseases elicited by abnormal platelet aggregation, diseases elicited by smooth muscle proliferation, Goodpasture syndrome, acute and chronic kidney diseases, glomerulonephritis, membranous nephropathy, diabetic nephropathy, neonatal pulmonary hypertension, asthma, congestive heart failure, adult pulmonary hypertension, renal vascular hypertension, proliferative retinopathies, experimental autoimmune encephalomyelitis, multiple sclerosis, insulin dependent diabetes, inflammatory bowel disease, ulcerative colitis, Crohn's disease, chronic inflammation, bone osteolysis, thrombosis, atherosclerosis, amyloidosis, hereditary multiple exostosis, bladder pain, vulvodynia, acute and chronic pancreatitis, empyema, diabetes, liver fibrosis, kidney fibrosis, peritoneal fibrosis (in particular, glucose induced following peritoneal dialysis) and aging.

A further object of the invention is a pharmaceutical composition containing as active ingredient a compound of formula (I) and at least one pharmaceutically acceptable vehicle and/or excipient.

### Detailed description of the invention

### Definitions

Within the meaning of the present invention, the term "anti-heparanase activity" refers to an activity sufficient to inhibit partially or totally the enzyme heparanase.

Within the meaning of the present invention, for "residue of an amino acid" it is intended the characterizing portion of an amino acid. Said portion is specific to each amino acid. The structure of the characterizing group for each known amino acid is commonly known in the art.

In the compounds of the invention, the group NHC=Y can be present on any free position of the phenyl ring to which it is attached. In particular, it can be in position 4 or 5. Preferably, it is in position 4 of the phenyl ring.

In the compounds of the invention, W is preferably fluorine (F).

In the compounds of the invention, Y is preferably selected from the group consisting of O and S.

In the compounds of the invention, Z is preferably selected from the group consisting of OH and NH-CHR-COOH wherein Rhas the meanings above defined.

When Z is NH-CHR-COOH, R can be the residue of any amino acid. In particular, it can be from a D-amino acid or a L-amino acid. Also, it can be from a natural or unnatural amino acid. The structure of the R group for each known amino acid is well known in the field. For example, the R group of glycine is H, the R group of alanine is CH₃, the R group of valine is CH(CH₃)₂, and so on.

In particular, R can be the residue of an amino acid selected from the group consisting of: alanine (Ala), arginine (Arg), asparagine (Asn), aspartic acid (Asp), cysteine (Cys), glutamic acid (Glu), glutamine (Gin), glycine (Gly), histidine (His), isoleucine (Ile), leucine (Leu), lysine (Lys), methionine (Met), phenylalanine (Phe), proline (Pro), serine (Ser), threonine (Thr), tryptophan (Trp), tyrosine (Tyr) and valine (Val).

Preferably, it is from an amino acid selected from the group consisting of: glycine (Gly), alanine (Ala) and phenylalanine (Phe). More preferably, R is a residue of glycine (Gly), therefore it is H.

When Z is NHCHR₂R₃, preferably R₂ and R₃ are not both H. In particular, in an embodiment, R₂ is CH₂CH(CH₃)₂ and R₃ is H; in an alternative embodiment, R₂ is H and R₃ is B(OH)₂; in another alternative embodiment, R₂ is CH₂CH(CH₃)₂ and R₃ is B(OH)₂.

In a preferred embodiment, compounds of the invention are those in which Y is O, X is NH and Z and W have the meanings above defined, W being preferably F. In particular, in this embodiment, Z is preferably NH-CHR-COOH, R being a residue of an amino acid preferably selected from Gly and Phe. In another embodiment, Z is NHCHR₂R₃, wherein R₂ is H and R₃ is B(OH)₂. In another preferred embodiment, Z is OH.

In another preferred embodiment, compounds of the invention are those in which Y is S, X is NH and W and Z have the meanings above defined, W being preferably F. In particular, in this embodiment, Z is preferably OH. In another preferred embodiment, Z is NH-CHR-COOH wherein R is preferably a residue of Gly.

In another preferred embodiment, compounds of the invention are those in which Y is O, X is O and W and Z have the meanings above defined, W being preferably F. In particular, in this embodiment, Z is preferably OH. In another preferred embodiment, Z is NH-CHR-COOH wherein R is a residue of an amino acid preferably selected from Gly, Ala and Phe. In another embodiment, Z is NHCHR₂R₃, wherein R₂ is CH₂CH(CH₃)₂ and R₃ is H, or R₂ is H and R₃ is B(OH)₂, or R₂ is CH₂CH(CH₃)₂ and R₃ is B(OH)₂.

In another preferred embodiment of the invention, compounds of the invention are those in which Y is S, X is O and W and Z have the meanings above defined, W being preferably F. In particular, in this embodiment, Z is preferably OH or NH-CHR-COOH.

In another embodiment of the invention, compounds of the invention are those in which Y is NH, X is NH and W and Z have the meanings above defined, W being preferably F. In particular, in a preferred embodiment Z is OH.

In another embodiment of the invention, compounds of the invention are those in which Y is NH, X is O and W and Z have the meanings above defined, W being preferably F.

Any nitrogen group can be protected. The protective group is preferably selected from tert-butoxycarbonyl, benzyloxycarbonyl and 9-fluorenylmethyloxycarbonyl.

When one or more chiral centers are present in the compounds of the present invention, the individual isomers, enantiomers and diastereoisomers, and mixtures thereof (e.g., racemates) are intended to be encompassed by the present invention.

The pharmaceutical acceptable salts of the compound of formula (I) are included in the scope of the invention.

Pharmaceutical acceptable salts are salts which retain the biological activity of the compound and are derived from known pharmacologically acceptable basis. Examples of pharmaceutically acceptable salts are, for example, those from compounds of formula (I) and inorganic bases, such as sodium, potassium, calcium and aluminum hydroxides, as well as those with organic bases such as, for example, lysine, arginine, N-methyl-glucamine, trimethylamine, triethanolamine, dibenzylamine, methylbenzylamine, di-(2-ethyl-hexyl)-amine, piperidine, N-ethylpiperidine, N,N-di-ethylaminoethylamine, N-ethylmorpholine, 3-phenethylamine, N-benzyl-3-phenethylamine, N-benzyl-N,N-dimethylamine. A preferred salt is sodium salt.

Also, pharmaceutically acceptable hydrates or solvates of the compound of formula (I) are included in the scope of the present invention. In particular, they can be any hydrate or solvate commonly used in the art.

Pharmaceutically acceptable salts and their preparation are within the common knowledge of the skilled person and general reference is made to the relevant literature, such as, for example Pharmaceutical Salts and Co-crystals, Johan Wouters, Luc Quéré, Royal Society of Chemistry, 2011.

Preferred compounds according to the present invention are the followings:
2,2'-(((Carbonylbis(azanediyl))bis(3-fluoro-4,1-phenylene))bis(benzo[d]oxazole-2,5-diyl))diacetic acid (20) (SST0578AA1),
2,2'-((2,2'-(((Carbonylbis(azanediyl))bis(3-fluoro-4,1-phenylene))bis(benzo[d]oxazole-2,5-diyl))bis(acetyl))bis(azanediyl))diacetic acid (25) (SST0795AA1),
(2S,2'S)-2,2'-((2,2'-(((Carbonylbis(azanediyl))bis(3-fluoro-4,1-phenylene))bis(benzo[d]oxazole-2,5-diyl))bis(acetyl))bis(azanediyl))dipropionic acid (27) (SST0796AA1),
(2S,2'S)-2,2'-((2,2'-(((Carbonylbis(azanediyl))bis(3-fluoro-4,1-phenylene))bis(benzo[d]oxazole-2,5-diyl))bis(acetyl))bis(azanediyl))bis(3-phenylpropanoic acid) (26) (SST0797AA1),
2,2'-(((Carbonylbis(azanediyl))bis(3-fluoro-4,1-phenylene))bis(1 H-benzo[d]imidazole-2,5-diyl))diacetic acid (4) (SST0861AA1),
(2S,2'S)-2,2'-((2,2'-(((Carbonylbis(azanediyl))bis(3-fluoro-4,1-phenylene))bis(1H-benzo[d]imidazole-2,5-diyl))bis(acetyl))bis(azanediyl))bis(3-phenylpropanoic acid) (8) (SST0866AA1),
2,2'-(((Thiocarbonylbis(azanediyl))bis(3-fluoro-4,1-phenylene))bis(1H-benzo[d]imidazole-2,5-diyl))diacetic acid (12) (SST0870AA1), and
2,2'-((2,2'-(((Thiocarbonylbis(azanediyl))bis(3-fluoro-4,1-phenylene))bis(benzo[d]oxazole-2,5-diyl))bis(acetyl))bis(azanediyl))diacetic acid (38) (SST0872AA1).

Further compounds according to the present invention are the followings:
2,2'-((2,2'-(((carbonylbis(azanediyl))bis(3-fluoro-4,1-phenylene))bis(1*H*-benzo[*d*]imidazole-2,5-diyl))bis(acetyl))bis(azanediyl))diacetic acid (7) - (SST0863AA1)
(((2,2'-(((Carbonylbis(azanediyl))bis(3-fluoro-4,1-phenylene))bis(1H-benzo[d]imidazole-2,5-diyl))bis(acetyl))bis(azanediyl))bis(methylene))diboronic acid (9) - (SST0880AA1)
2,2'-(((Carbonylbis(azanediyl))bis(3-fluoro-4,1-phenylene))bis(benzo[d]oxazole-2,5-diyl))bis(N-isopentylacetamide) (28) - (SST0862AA1)
(1R,1'R)-(((2,2'-(((Carbonylbis(azanediyl))bis(3-fluoro-4,1-phenylene))bis(benzo[d]oxazole-2,5-diyl))bis(acetyl))bis(azanediyl))bis(3-methylbutane-1,1-diyl))diboronic acid (31) - (SST0878AA1),
2,2'-(((Carbonylbis(azanediyl))bis(4,1-phenylene))bis(benzo[d]oxazole-2,5-diyl))diacetic acid (19) - (SST0564AA1)
2,2'-(((thiocarbonylbis(azanediyl))bis(4,1-phenylene))bis(benzo[d]oxazole-2,5-diyl))diacetic acid (33) - (SST0587AA1)
2,2'-(((Thiocarbonylbis(azanediyl))bis(3-fluoro-4,1-phenylene))bis(benzo[d]oxazole-2,5-diyl))diacetic acid (36) - (SST0626AA1) and
2,2'-((((Iminomethylene)bis(azanediyl))bis(4,1-phenylene))bis(benzo[d]oxazole-2,5-diyl))diacetic acid (40) (SST0665AA1).

The compounds of the present invention can be synthesized by many methods available to those skilled in the art of organic chemistry. General and exemplary synthetic schemes for preparing compounds of the present invention are described below. These schemes are illustrative and are not meant to limit the possible techniques one skilled in the art may use to prepare the compounds disclosed herein. Different methods to prepare the compounds of the present invention will be evident to those skilled in the art. Additionally, the various steps in the synthesis may be performed in an alternate sequence in order to give the desired compound or compounds.

Examples of compounds of the present invention prepared by methods described in the general schemes are given in the intermediates and examples section set out hereinafter.

The compounds of the present invention can be synthesized using the methods described below, together with synthetic methods known in the art of synthetic organic chemistry, or by variations thereon as appreciated by those skilled in the art. Preferred methods include, but are not limited to, those described below. The reactions are performed in a solvent or solvent mixture appropriate to the reagents and materials employed and suitable for the transformations being effected. It will be understood by those skilled in the art of organic synthesis that the functionalities present on the molecule should be consistent with the transformations proposed.

It will also be recognized that another major consideration in the planning of any synthetic route in this field is the judicious choice of the protecting group used for protection of the reactive functional groups present in the compounds described in this invention. An authoritative account describing the many alternatives to the trained practitioner is Greene et al. (Protective Groups in Organic Synthesis, 4th Edition, Wiley- Interscience (2006)).

Also, the skilled in the art can easily alter the reagents and reaction conditions exemplified in the schemes below to include any combination of substituents encompassed by the present invention. Also, the skilled artisan can easily use interchangeable steps for each synthetic process and incorporate isolation and/or purification steps as deemed necessary.

All the conditions and reagents not explicitly mentioned below can be easily chosen by the skilled in the art according to the general knowledge in the field.

Starting materials useful for the preparing the compounds of the invention and intermediates therefor are commercially available or can be prepared by well known synthetic methods.

The final products obtained by the synthesis described below may be purified using techniques commonly known to one skilled in the art such as preparatory chromatography, thin-layer chromatography, HPLC, or crystallization.

Exemplary processes for the synthesis of the compounds of the invention are herein described.

### Exemplary processes

### Benzimidazoles

Exemplary processes to obtain compounds according to the present invention wherein X is NH are herein disclosed.

In particular, an exemplary process for obtainment of compounds of formula (I) wherein W is F, Y is O, X is NH and Z has the meanings above defined is herein disclosed.

A first exemplary procedure is represented in the following Scheme 1.

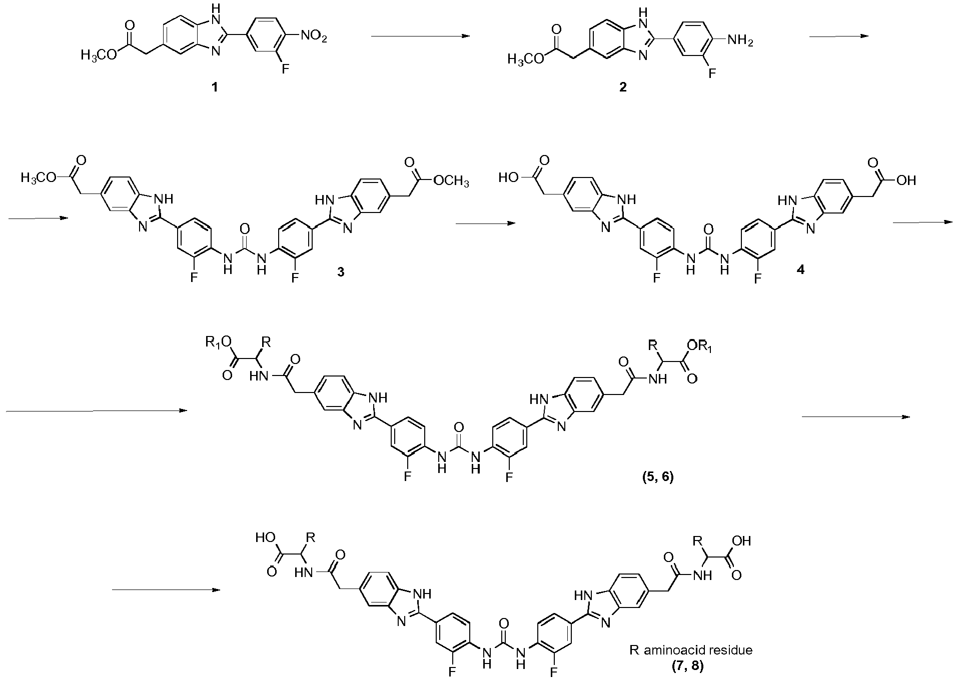

Scheme 1. R represents an amino acid residue, R₁ represents a protective group.

Compound 2 is obtained by reduction of methyl 2-(2-(3-fluoro-4-nitrophenyl)-1*H-*benzo[*d*]imidazol-5-yl)acetate (1) (obtained according to Bahrami, K. et al, J. Org. Chem. 2008, 73(17), 6835 from the suitable starting material) using common reduction means, for example diethylamine and ammonium formate. Palladium on carbon (Pd/C) can be used as a catalyst; the reaction is carried out in suitable solvents, for example ethyl acetate. Compound 3 is then obtained by adding a condensation agent, for example triphosgene, to the obtained compound 2. Triethylamine can be used in the reaction. The terminal alkyl ester of compound 3 is hydrolysed with a suitable reagent, for example with lithium hydroxide, and compound 4 (Z = OH) is obtained. Reactions can be carried out in suitable solvents, for example tetrahydrofuran.

To obtain compounds wherein Z comprises an amino acid residue, the desired amino acid is added, usually in esterified form, for example as commercially available amino ester hydrochloride, and compounds 5,6 are obtained, wherein R₁ is a protective group, for example an alkyl, such as methyl or ethyl. The reaction can be carried out in presence of suitable reagents, for example catalysts or activators, such as 1-ethyl-3-(3-dimethylaminopropyl)carbodiimide (EDCI), 4-dimethylaminopyridine (DMAP) and N,N-Diisopropylethylamine (DIPEA), in a suitable solvent, such as N,N-dimethylformamide (DMF). Finally, the ester present on the amino acid residue is hydrolysed using a suitable reagent, for example LiOH, to obtain the desired amino acid derivative (compounds 7, 8).

R can be any amino acid residue according to the present invention.

Similar compounds wherein W is H can be easily obtained by the procedure above described using commercially available methyl 2-(2-(4-nitrophenyl)-1*H*-benzo[*d*]imidazol-5-yl)acetate as starting compound instead of compound 1.

Compounds wherein Z is NHCHR₂R₃, wherein R₂ is H and R₃ is B(OH)₂ can be obtained starting from the above mentioned compound 4 and adding commercially available (4,4,5,5-tetramethyl-1,3,2-dioxaborolan-2-yl)methanaminium chloride, as represented in the following scheme 2. The reaction can be carried out using further reagents, such as N,N,N',N'-tetramethyl-O-(3,4-dihydro-4-oxo-1,2,3-benzotriazin-3-yl)uronium tetrafluoroborate (TBTU), in suitable solvents, such as tetrahydrofuran and N,N-dimethylformamide.

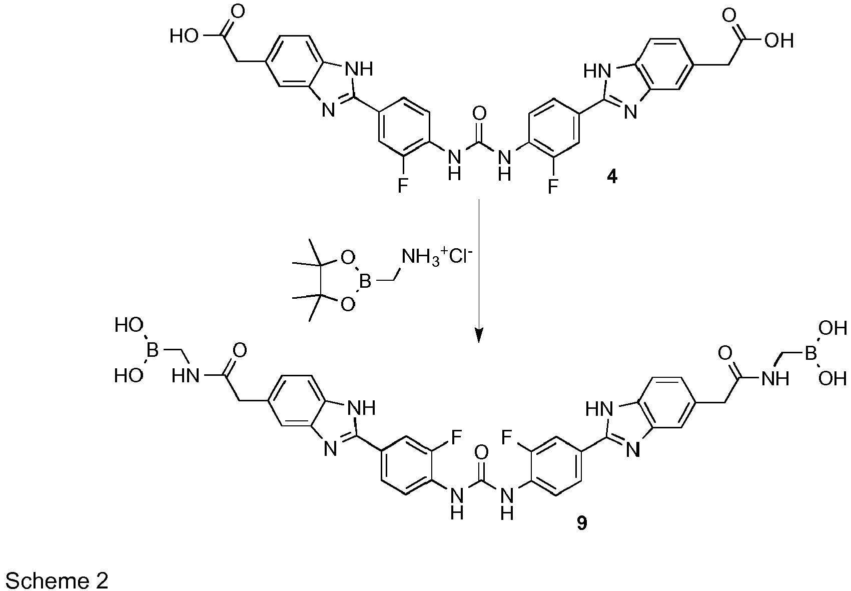

Compounds wherein Z is NHCHR₂R₃, wherein R₂ is CH₂CH(CH₃)₂ and R₃ is B(OH)₂ can be obtained starting from the above mentioned compound 4 and adding commercially available (R)-boroLeu-(+)-pinanediol HCl and suitable reagents, for example O-(benzotriazol-1-yl)-N,N,N',N'-tetramethyluronium tetrafluoroborate (TBTU) and N,N-Diisopropylethylamine (DIPEA), in suitable solvents, for example N,N-dimethylformamide and/or tetrahydrofuran, at a temperature preferably comprised between -60°C and -100°C, preferably being of about - 80°C, to obtain an intermediate boronic ester, which is then deprotected, for example with ammonium acetate (NH₄OAc_{Aq}) and sodium periodate (NalO₄), to obtain compounds according to the invention.

Compounds wherein Z is NHCHR₂R₃, wherein R₂ is CH₂CH(CH₃)₂ and R₃ is H can be obtained starting from the above mentioned compound 4 and adding commercially available isopentylamine and suitable coupling reagents, for example 1-ethyl-3-(3-dimethylaminopropyl)carbodiimide (EDCI), (benzotriazol-1-yl-oxytripyrrolidinophosphonium hexafluorophosphate) (PyBOP), 4-Dimethylaminopyridine (DMAP) and/or N,N-Diisopropylethylamine (DIPEA), in suitable solvents, for example tetrahydrofuran, DCM (Dichloromethane) or DMF (N,N-dimethylformamide) preferably at room temperature.

Alternatively, compounds wherein Z is NHCHR₂R₃, wherein R₂ is CH₂CH(CH₃)₂ and R₃ is H can be obtained starting from the above mentioned compound 4 and adding commercially available (R)-boroLeu-(+)-pinanediol HCl and suitable reagents, for example 1-ethyl-3-(3-dimethylaminopropyl)carbodiimide (EDCI), 4-dimethylaminopyridine (DMAP) and/or N,N-diisopropylethylamine (DIPEA), in suitable solvents, for example tetrahydrofuran, preferably at room temperature.

An exemplary process for obtainment of compounds of formula (I) wherein W is F, Y is S, X is NH and Z can have any of the meanings above defined is herein disclosed.

An exemplary procedure is represented in the following Scheme 3.

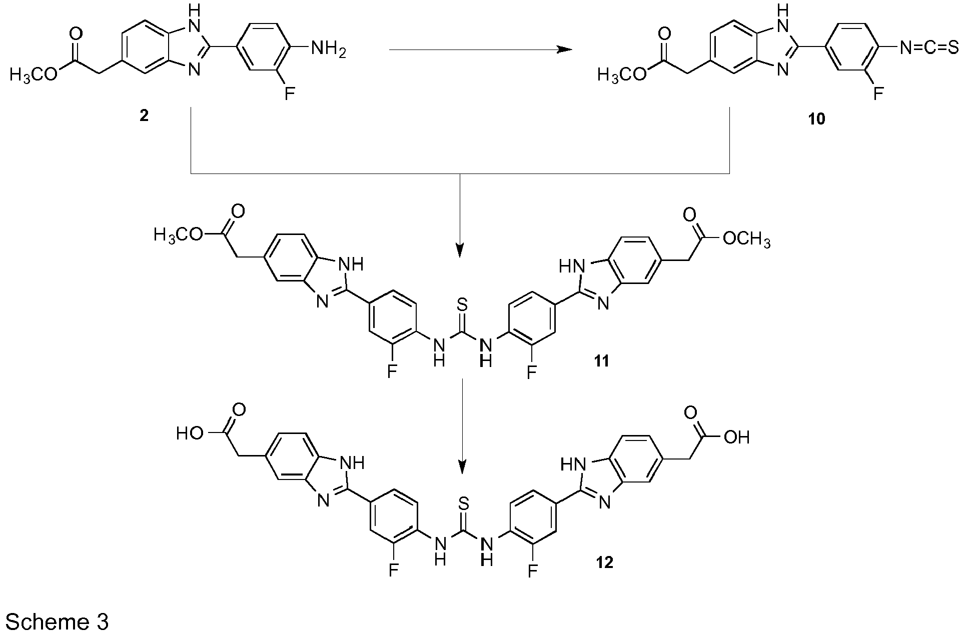

Compound 10 is obtained by reaction of the above mentioned compound 2 with thiophosgene in suitable solvents, such as for example chlorobenzene and DMF, preferably at room temperature. By mixing the obtained compound 10 with compound 2 in a suitable solvent, such as dimethylsulfoxide (DMSO), compound 11 is obtained. For example, microwave at about 80°C for about 3 min under argon atmosphere can be used. The terminal ester of compound 11 is then hydrolysed using a suitable reagent, for example NaOH, to obtain the desired derivative (compound 12, Z = OH).

Compounds wherein Z comprises an amino acid residue or is NHCHR₂R₃ can be obtained by adding the suitable compounds to the obtained derivative 12, as above disclosed and exemplified for compounds wherein Y is O.

Similar compounds wherein W is H can be easily obtained from the same procedure above described using commercially available methyl 2-(2-(4-nitrophenyl)-1*H*-benzo[*d*]imidazol-5-yl)acetate as starting compound instead of compound 1.

### Benzoxazoles

Exemplary processes to obtain compounds according to the present invention wherein X is O are herein disclosed.

In particular, an exemplary process for obtainment of compounds of formula (I) wherein Y is O, X is O and W and Z can have any of the meanings above defined is disclosed in the following.

In particular, an exemplary process wherein Z is OH is represented in the following Scheme 4.

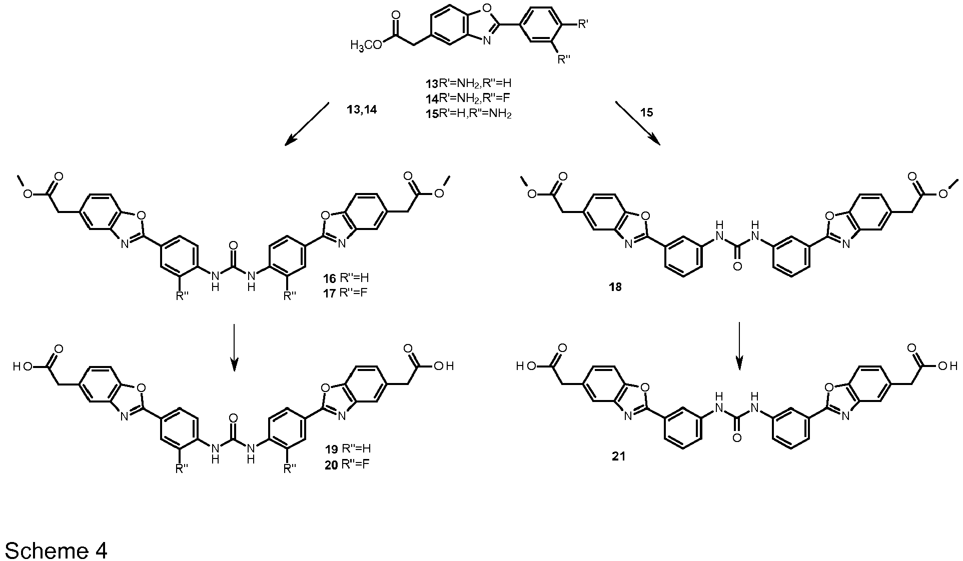

A condensation agent, for example triphosgene, is added to the suitable amine starting compound 13-15. Said amine starting compounds are all commercially available or they are obtainable according to WO2004046122 from suitable starting materials. Corresponding urea derivatives 16-18 are obtained. The reaction can be carried out using further suitable reagents, such as trimethylamine, in suitable solvents such as dichloromethane (DCM). The terminal ester is then hydrolysed using a suitable reagent, for example LiOH, to obtain the desired compound 19-21 (Z = OH).

Depending on the starting compound, final compounds wherein W is H or F can be obtained.

Compounds wherein the urea group is linked on different positions of the phenyl ring can be obtained by choosing the suitable starting compound. Exemplary cases wherein the urea group is linked on position 4 or 5 of the phenyl ring are shown in the above scheme 4.

Compounds wherein Z comprises an amino acid residue or is NHCHR₂R₃ can be obtained by adding the suitable compounds to the obtained urea derivative 19-21, as already described above.

For example, as represented in the following scheme 5, when Z comprises an amino acid residue, the suitable amino ester hydrochloride can be added to compound 19-21 to obtain the corresponding amide derivatives (22-24), as already above described. Further suitable reagents can be used, such as EDCI, DMAP and DIPEA, in suitable solvents, such as THF, as already above described. The ester function on the amino acid residue is then hydrolysed using a suitable reagent, for example LiOH, to obtain the desired final derivative (compounds 25-27).

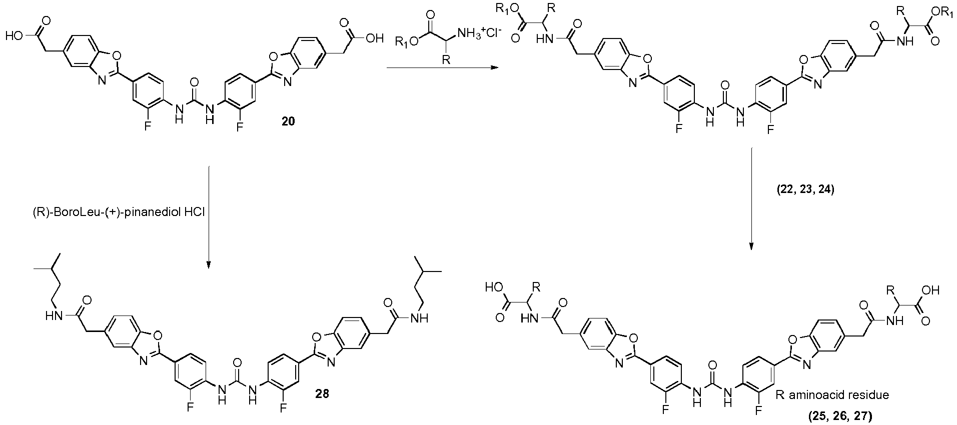

Scheme 5. R is an amino acid residue, R₁ is a protective group, for example an alkyl group, such as methyl or ethyl.

Compounds wherein Z is NHCHR₂R₃ can be obtained, for example, through the following processes, depending on the desired R₂ and R₃.

For example, a compound (28) wherein R₂ is CH₂CH(CH₃)₂ and R₃ is H can be obtained starting from the above mentioned compound 20 and adding commercially available isopentylamine and suitable coupling reagents, for example 1-ethyl-3-(3-dimethylaminopropyl)carbodiimide (EDCI), (benzotriazol-1-yloxy)tripyrrolidinophosphonium hexafluorophosphate (PyBOP), 4-dimethylaminopyridine (DMAP) and/or N,N-diisopropylethylamine (DIPEA), in suitable solvents, for example tetrahydrofuran, DCM or DMF preferably at room temperature.

Alternatively, compounds wherein Z is NHCHR₂R₃, wherein R₂ is CH₂CH(CH₃)₂ and R₃ is H can be obtained starting from the above mentioned compound 20 and adding commercially available (R)-boroLeu-(+)-pinanediol HCl and suitable reagents, for example 1-ethyl-3-(3-dimethylaminopropyl)carbodiimide (EDCI), 4-dimethylaminopyridine (DMAP) and N,N-diisopropylethylamine (DIPEA), in suitable solvents, for example tetrahydrofuran, preferably at room temperature, as shown in the above scheme 5.

Compounds wherein R₂ is H and R₃ is B(OH)₂ can be obtained by adding to compound 20 (4,4,5,5-tetramethyl-1,3,2-dioxaborolan-2-yl)methanaminium chloride and suitable reagents, for example O-(benzotriazol-1-yl)-N,N,N',N'-tetramethyluronium tetrafluoroborate (TBTU), and N,N-diisopropylethylamine (DIPEA), in suitable solvents, for example N,N-dimethylformamide, at a temperature typically comprised between -60°C and -100°C, preferably being about -80°C, as represented in the scheme 6 below.

Compounds wherein R₂ is CH₂CH(CH₃)₂ and R₃ is B(OH)₂ can be obtained by adding to compound 20 (R)-boroLeu-(+)-pinanediol HCl and suitable reagents, for example O-(benzotriazol-1-yl)-N,N,N',N'-tetramethyluronium tetrafluoroborate (TBTU) and N,N-diisopropylethylamine (DIPEA), in suitable solvents, for example N,N-dimethylformamide at a preferred temperature comprised between -60°C and -100°C, preferably being about -80°C, to obtain the boronate ester 29, to which suitable reagents such as NH₄OAc and NalO₄ are added to obtain final compound 31, as represented in the following scheme 6.

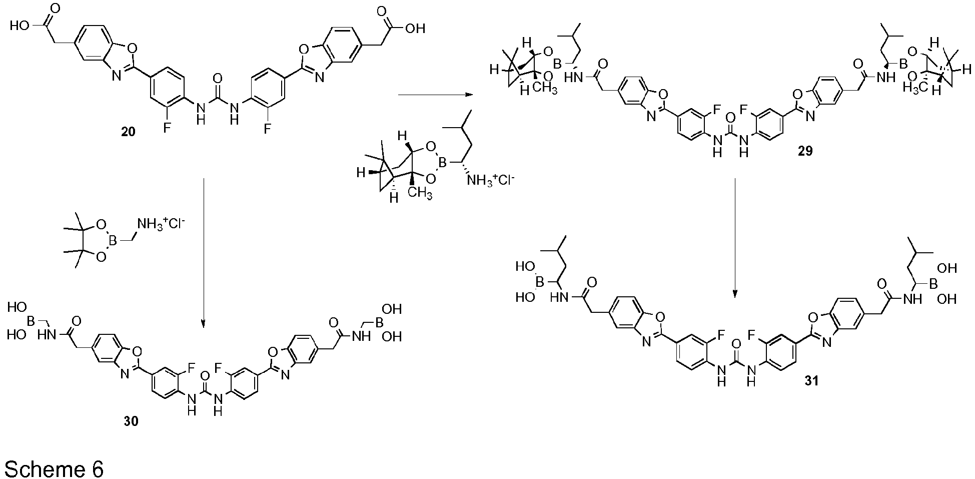

Similar processes can be used to obtain boronic derivatives of any compound according to formula (I) by choosing the suitable starting compound.

For example, compounds wherein W is H can be easily obtained with the same procedure above described using compound 19 (W=H) as starting compound instead of compound 20.

An exemplary process for obtainment of compounds of formula (I) wherein Y is S, X is O and W and Z can have any of the meanings above defined is disclosed in the following.

In particular, a process wherein Z is OH is shown in the scheme 7 below.

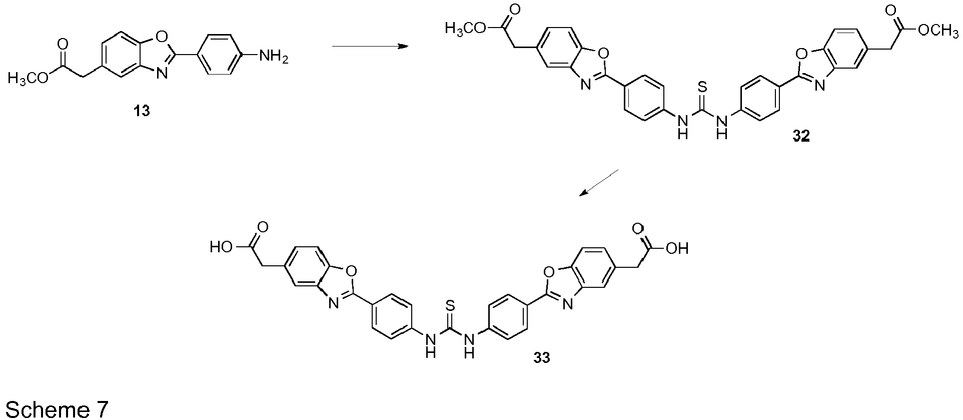

To the above mentioned amine starting compound 13, N,N'-thiocarbonyldiimidazole is added to obtain compound 32, wherein terminal esters are then hydrolyzed using a suitable base, for example NaOH, to obtain final compound 33 (Z = OH).

Similar compounds wherein W is F can be easily obtained with the same procedure above described using the suitable amine starting compound, for example compound 14 above described.

Alternatively, the exemplary process shown in the scheme 8 below can be used.

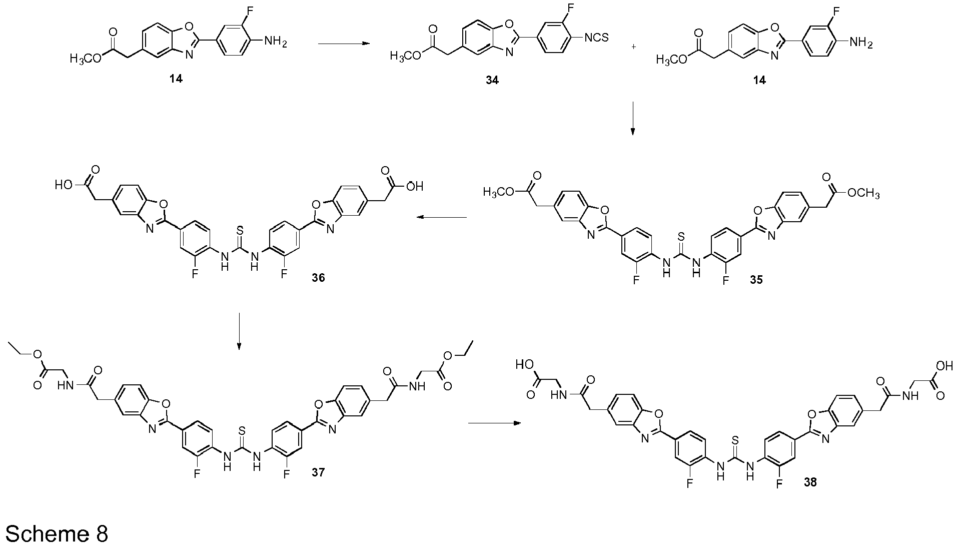

Methyl 2-(2-(4-amino-3-fluorophenyl)benzo[d]oxazol-5-yl)acetate 14 is reacted with thiophosgene to obtain compound 34, which is then reacted with compound 14 in suitable conditions, for example using microwave at about 80°C for about 3 min under argon atmosphere, to obtain compound 35. The terminal ester is then hydrolyzed using a suitable base, for example NaOH, thus obtaining compound 36 (Z = OH).

Compounds wherein Z is an amino acid or is NHCHR₂R₃ can be obtained by a procedure similar to the one already described above by adding the suitable compounds to the obtained compound 36. For example, as represented in the above scheme 8, when Z is an amino acid, the suitable amino ester hydrochloride can be added to compound 36 to obtain the amide derivative 37. The ester function of the amino acid is then hydrolysed using a suitable reagent, for example NaOH, to obtain the desired derivative 38. In scheme 8, Z is a glycine residue but the same process can be used with any amino acid adding the corresponding amino ester hydrochloride to compound 36.

An exemplary process for obtainment of compounds of formula (I) wherein Y is NH, X is O and W and Z can have any of the meanings above defined is disclosed in the following.

In particular, an exemplary process wherein Z = OH is shown in scheme 9 below.

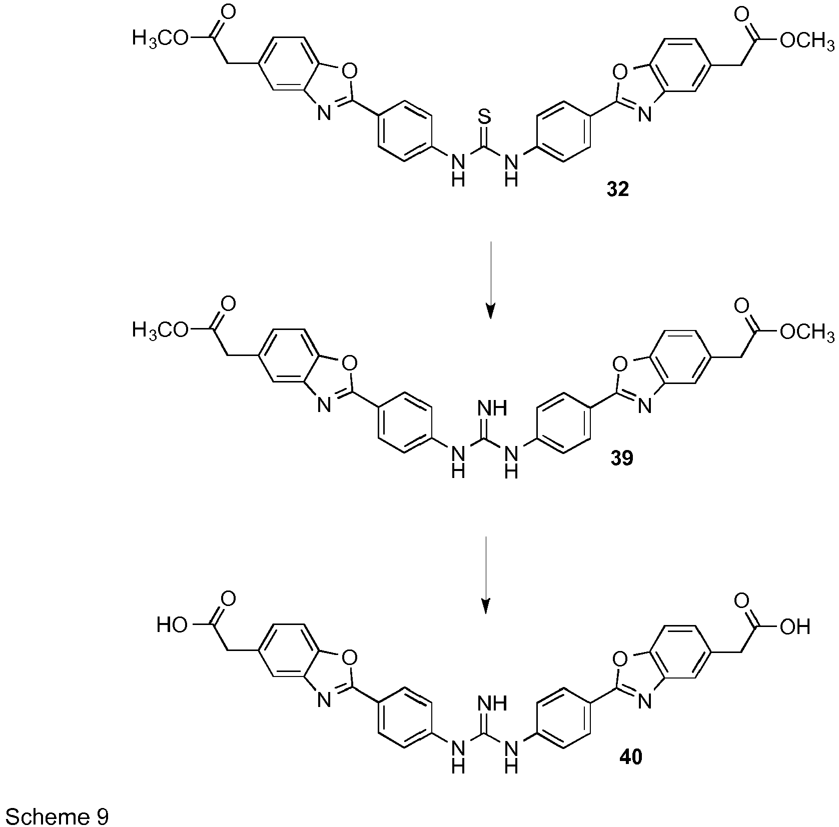

With reference to above scheme 9, commercially available hexamethyldisilazane is added to compound 32, which can be obtained as above described, preferably in presence of 1-ethyl-3-(3-dimethylaminopropyl)carbodiimide (ECDI), to obtain guanidine compound 39. The terminal ester is then hydrolyzed using a suitable base, for example LiOH, thus obtaining compound 40 according to the invention (Z = OH).

Similar compounds wherein W is F can be easily obtained with the same procedure above described using the suitable amine starting compound, for example compound 14, as shown above.

Compounds wherein Z comprises an amino acid residue or is NHCHR₂R₃ can be obtained by a procedure similar to the one above described, by adding the suitable compounds to the obtained guanidine derivative 40, according to general procedures already described above.

Compounds of formula (I) wherein Y is NH, X is NH and W and Z can have any of the meanings above defined can be obtained with the same procedure above described and shown in scheme 9 but starting from compound 12 instead of compound 32.

All the transformations above described are only exemplary synthetic procedures already known in organic chemistry and well known to the experts in the field.

Any variation or combination of substituents of compounds of formula (I) which is within the scope of the present invention and is not explicitly shown or described in the above processes, can be easily obtained starting from the exemplary processes described above with suitable modifications, for example in the starting compounds or reagents, that are well within the knowledge of the skilled person in the field of organic chemistry. Reference can be made, for example, to the handbook "March's Advanced Organic Chemistry, Reactions, Mechanism and structures.", Michael B. Smith, Jerry March, last edition.

### Medical uses

According to the present invention, the compounds of formula (I) can be used as medicaments.

In particular, according to the present invention, the compounds of formula (I) are endowed with anti-heparanase activity. Therefore, they can be advantageously used for the treatment of diseases which can be improved or prevented by the inhibition of heparanase activity.

The heparanase has a critical role in modulating the microenvironment via the ECM (extracellular matrix) remodeling and thus influencing a multitude of both normal and disease-related processes, as inflammation, blood coagulation, wound healing, angiogenesis, fibrosis and tumor cell growth, invasion and metastasis (for a review of the roles and activities of heparanase see for example "Heparanase: From basic research to therapeutic applications in cancer and inflammation", Vlodavsky I., Singh P., Boyango I., Gutter-Kapon L., Elkin M., Sanderson RD, Ilan N., Drug Resist. Updat. 2016, 29, 54). Heparanase has also been recently reported to play a fundamental role in many other pathologies, such as nephropathies of different origin, severe infective disorders, gastrointestinal diseases, osteolysis in bone metastases as well as in other bone tissue related pathologies, atherosclerosis, cardiovascular disorders and cutaneous aging. The panorama of diseases affected by heparanase expression and activity also includes the so-called rare diseases, such as amyloidosis, hereditary multiple exostoses and idiopathic avascular necrosis of bone as well as pathologies that are not rare in terms of incidence, but are rarely diagnosed, such as vulvodynia (Rivara et al., 2016).

Therefore, the compounds of formula (I) can be advantageously used in a broad range of diseases.

In particular, heparanase expression is enhanced in cancers, including various carcinomas, sarcomas and hematological malignancies, and it has been demonstrated that it correlates with increased tumor size, tumor angiogenesis, enhanced metastasis and poor prognosis. Indeed, treatments of tumor-bearing mice with heparanase-inhibiting compounds has been shown to markedly attenuate tumor progression further supporting an anti-heparanase therapy for multiple types of cancer (Vlodavski et al., 2016).

Therefore, the compounds of the invention, thanks to their demonstrated activity as heparanase inhibitors, can be advantageously used in the treatment of tumors and metastasis.

In particular, they can be used in the treatment of any kind of tumor, both solid and liquid. For example they can be used in the treatment of solid tumors, such as for example glioma and sarcomas, such as fibrosarcoma and osteosarcoma, and in the treatment of hematological malignancies.

The involvement of heparanase in inflammatory reactions has also been shown (Vlodavski et al., 2016) thus supporting the use of inhibitors of heparanase also in diseases characterized by or related with acute and chronic inflammation, such as colitis, atherosclerosis, pancreatitis, Chron's disease, psoriasis and others.

More recently, heparanase overexpression has been documented to be related to the endothelial mesenchymal transition (EMT; Masola V. et al. Specific heparanase inhibition reverses glucose-induced mesothelial-to-mesenchymal transition Nephrol. Dial. Transplant 2017, 1) and thus heparanase inhibition may be also beneficial in those pathologic processes involving EMT, as fibrosis occurring at different organs.

Examples of diseases which can be treated by compounds of formula (I) are tumors, primary tumors, metastases, diabetic retinopathies, psoriasis, retrolenticular fibroplasia, restenosis after angioplasty, coronary by-pass, inflammation, sepsis, arthritis, autoimmune diseases, allograft rejection, cardiovascular diseases, fibro-proliferative disease, diseases elicited by abnormal platelet aggregation, diseases elicited by smooth muscle proliferation, Goodpasture syndrome, acute and chronic kidney diseases, glomerulonephritis, membranous nephropathy, diabetic nephropathy, neonatal pulmonary hypertension, asthma, congestive heart failure, adult pulmonary hypertension, renal vascular hypertension, proliferative retinopathies, experimental autoimmune encephalomyelitis, multiple sclerosis, insulin dependent diabetes, inflammatory bowel disease, ulcerative colitis, Crohn's disease, chronic inflammation, bone osteolysis, thrombosis, atherosclerosis, amyloidosis, hereditary multiple exostosis, bladder pain, vulvodynia, acute and chronic pancreatitis, empyema, diabetes, liver fibrosis, kidney fibrosis, peritoneal fibrosis (in particular, peritoneal fibrosis glucose induced following peritoneal dialysis) and aging.

The skilled in the art, for example a physician, can identify and recognize if a disease can be ameliorated by an anti-heparanase activity on the basis of the common knowledge in the field. For example, for a broad, even though not exhaustive, overview, on possible clinical applications of heparanase inhibitors, reference can be made to the reviews of Rivara et al., 2016 and Vlodavski et al., 2016.

Also, tests are known in the field and available to the skilled person to evaluate if a compound is endowed with an anti-heparanase activity, for example the AGAIA assay (Hammond E. et al. Anal. Biochem. 2010, 396, 112).

Compounds of the invention are particularly advantageous also for non-tumoral applications since they are endowed with a strong anti-heparanase activity and therefore they can already be efficiently used at very low doses at which they are not cytotoxic. Indeed, in some embodiments they are selective heparanase inhibitors already at concentrations in which they do not interfere significantly with cell proliferation. This renders them particularly useful for therapeutic applications wherein a cytotoxic effect is undesired. Also, it has been found that such compounds at not cytotoxic concentrations are already able to inhibit invasion of tumor cells thus being particularly useful in the treatment of tumors and metastasis, also as adjuvants of chemotherapeutic agents. The compounds of the invention are also able to interfere with adhesion properties of cancer cells and they have an inhibitory effect against tumor transcription genes encoding for proangiogenic factors thus further supporting their therapeutic use in cancer diseases and metastasis.

The skilled person in the field knows how to determine the suitable dose in view of the desired application and his general knowledge in the medical and pharmaceutical field and by carrying out routine experimentation.

Another object of the present invention is a pharmaceutical composition containing at least one compound of formula (I) as an active ingredient, preferably in an amount such as to produce a significant therapeutic effect, together with pharmaceutically acceptable vehicles and/or excipients.

Such pharmaceutical compositions can be prepared by conventional methods and techniques which are common practice in the pharmaceutical industry, such as, for example, those illustrated in Remington's Pharmaceutical Science Handbook, Mack Pub. N.Y. - last edition.

The compositions according to the present invention contain, along with the active ingredient, at least one pharmaceutically acceptable vehicle or excipient. These may be particularly useful formulation coadjuvants, e.g. solubilising agents, dispersing agents, suspension agents, and emulsifying agents.

The pharmaceutical composition according to the invention comprising the compound of formula (I) can also contain one or more further active ingredients, for example chemotherapeutic agents. In particular, when the pharmaceutical composition is used for the treatment of a tumor, it preferably comprises also a chemotherapeutic agent. Indeed in some embodiments, the compounds of the invention act by modifying the tumor microenvironment to affect cell growth and spread as well as to facilitate the tumor killing action of other agents. Therefore, the combination of the compounds of the invention with any chemotherapeutic agent can be particularly useful. The chemotherapeutic agent can be chosen among the known agents according to the general knowledge of the skilled person in the field. For example, said chemotherapeutic agent can be selected from the group consisting of cytotoxic agents, proteasome inhibitors, immunomodulatory drugs, topoisomerase inhibitors, kinase inhibitors and monoclonal antibodies, in particular directed towards highly expressed tumor associated antigens. When said chemotherapeutic agent is a cytotoxic agent, it is preferably selected from the group consisting of melphalan, irinotecan, rituximab and lapatinib. When said chemotherapeutic agent is a proteasome inhibitor, it is preferably selected from the group consisting of bortezomib, carfilzomib and ixazomib. When said chemotherapeutic agent is an immunomodulatory agent, it is preferably selected from the group consisting of thalidomide, lenalidomide and pomalidomide.

For example, when the disease to be treated is multiple myeloma (MM), the compounds of the invention can be administered in conjunction with chemotherapy drugs known to be effective in MM, such as for example cytotoxic agents, for example melphalan, proteasome inhibitors, immunomodulatory drugs (IMIDs) and monoclonal antibodies towards highly expressed myeloma associated antigens.

According to the administration route chosen, the compositions will be in solid or liquid form, suitable for oral, parenteral or topical administration.

Generally, the compounds of this invention are administered in a "therapeutically effective amount". The amount of the compound actually administered will typically be determined by a physician, in the light of the relevant circumstances, including the condition to be treated, the chosen route of administration, the actual compound administered, drug combination, the age, body weight, and response of the individual patient, the severity of the patient's symptoms, and the like. For any compound, the therapeutically effective dose can be estimated initially either in cell culture assays or in animal models, usually mice, rats, guinea pigs, rabbits, dogs, or pigs. The animal model may also be used to determine the appropriate concentration range and route of administration. Such information can then be used to determine useful doses and routes for administration in humans. In calculating the Human Equivalent Dose (HED) it is recommended to use the conversion table provided in Guidance for Industry and Reviewers document (2002, U.S. Food and Drug Administration, Rockville, Maryland, USA).

Generally, an effective dose will be from 0.01 mg/kg to 100 mg/kg, preferably 0.05 mg/kg to 50 mg/kg. For any compound, the therapeutically effective dose can be estimated initially either in cell culture assays or in animal models, usually mice, rats, guinea pigs, rabbits, dogs, or pigs. The precise effective dose for a human subject will depend upon the specific disease to be treated, the severity of the disease state, general health of the subject, age, weight, and gender of the subject, diet, time and frequency of administration, drug combination(s), reaction sensitivities, and tolerance/response to therapy. This amount can be determined by routine experimentation and is within the judgement of the clinician.

Compositions may be administered individually to a patient or may be administered in combination with other agents, drugs or hormones. For example they can be administered in combination with further active ingredients, such as for example anticancer drugs.

The medicament may also contain a pharmaceutically acceptable carrier, for administration of a therapeutic agent. Such carriers include antibodies and other polypeptides, genes and other therapeutic agents such as liposomes, provided that the carrier does not induce the production of antibodies harmful to the individual receiving the composition, and which may be administered without undue toxicity.

Suitable carriers may be large, slowly metabolized macromolecules such as proteins, polysaccharides, polylactic acids, polyglycolic acids, polymeric amino acids, amino acid copolymers and inactive virus particles.

A thorough discussion of pharmaceutically acceptable carriers is available in Remington's Pharmaceutical Sciences (Mack Pub. Co., N.J. 1991).

Pharmaceutically acceptable carriers in therapeutic compositions may additionally contain liquids such as water, saline, glycerol and ethanol.

Additionally, auxiliary substances, such as wetting or emulsifying agents, pH buffering substances, and the like, may be present in such compositions. Such carriers enable the pharmaceutical compositions to be formulated as tablets, pills, dragees, capsules, liquids, gels, syrups, slurries, suspensions, and the like, for ingestion by the patient.

Once formulated, the compositions of the invention can be administered directly to the subject. The subjects to be treated can be animals; in particular, human subjects can be treated.

The medicament of this invention may be administered by any number of routes including, but not limited to, oral, intravenous, intramuscular, intra-arterial, intramedullary, intrathecal, intraventricular, transdermal or transcutaneous applications, subcutaneous, intraperitoneal, intranasal, enteral, topical, sublingual, intravaginal or rectal means.

The compositions for oral administration may take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include refilled, pre-measured ampoules or syringes of the liquid compositions or pills, tablets, capsules or the like in the case of solid compositions. In such compositions, the compound of the invention is usually a minor component (from about 0.1 to about 50% by weight or preferably from about 1 to about 40% by weight) with the remainder being various vehicles or carriers and processing aids helpful for forming the desired dosing form. Dosage treatment may be a single dose schedule or a multiple dose schedule.

A further object of the invention is a process for the preparation of pharmaceutical compositions characterized by mixing one or more compounds of formula (I) with suitable excipients, stabilizers and/or pharmaceutically acceptable diluents.

It is also an object of the invention the use of such pharmaceutical compositions as a medicament, in particular in the treatment of a disease which can be improved or prevented by an anti-heparanase activity. More in particular, the above mentioned diseases can be treated by such pharmaceutical compositions.

In an embodiment, the compounds of the invention can also be used for cosmetic applications thanks to their activity of inhibition of heparanase. Indeed, heparanase is produced in the skin by epidermal keratinocytes composing the epidermis and fibroblasts or vascular endothelial cells of the dermis and it decomposes heparan sulfate proteoglycan, which regulates differentiation and growth of the epidermis. It is known that heparanase can promote the formation of wrinkles; indeed, inhibition of heparanase activity suppresses the release of growth factors that accompanies decomposition of heparan sulfate, and allows migration of growth factors between the epidermis and dermis to be controlled, thereby aiding in anti-aging and anti-wrinkles effects of the skin (see for example EP2484349, US2014080878 and the work "Hyperpigmentation in human solar lentigo is promoted by heparanase-induced loss of heparan sulfate chains at the dermal-epidermal junction." Iriyama S., Ono T., Aoki H., Amano S. Dermatol Sci. 2011, 64(3), 223).

In view of the above and of the heparanase inhibition activity of the compounds of the invention, it is also an object of the present invention the cosmetic use of the compounds of formula (I) for prevention or treatment of skin aging. In particular, they can be used for preventing or suppressing the formation of wrinkles, in particular wrinkles caused by reduced level of heparanase sulfate in the epidermal basal membrane.

The compounds of the invention are particularly suitable for such cosmetic applications due to their low cytotoxicity. In particular, due to their potent anti-heparanase activity, they can already exert their effect at very low doses, at which they are not cytotoxic and thus suitable for cosmetic uses.

It is also an object of the invention, a cosmetic composition comprising the compound of formula (I) and cosmetically acceptable excipients and/or vehicles. Such excipients or vehicles can be chosen by the skilled person among the cosmetic ingredients commonly used in the field. In particular, they are components commonly used in external preparations. Such cosmetic composition can be used in the prevention or suppression of wrinkles or in any other anti-aging applications. It is preferably applied and administered topically.

The following examples further illustrate the invention.

### EXAMPLES

### Example 1

### Compounds 4, 7, 8

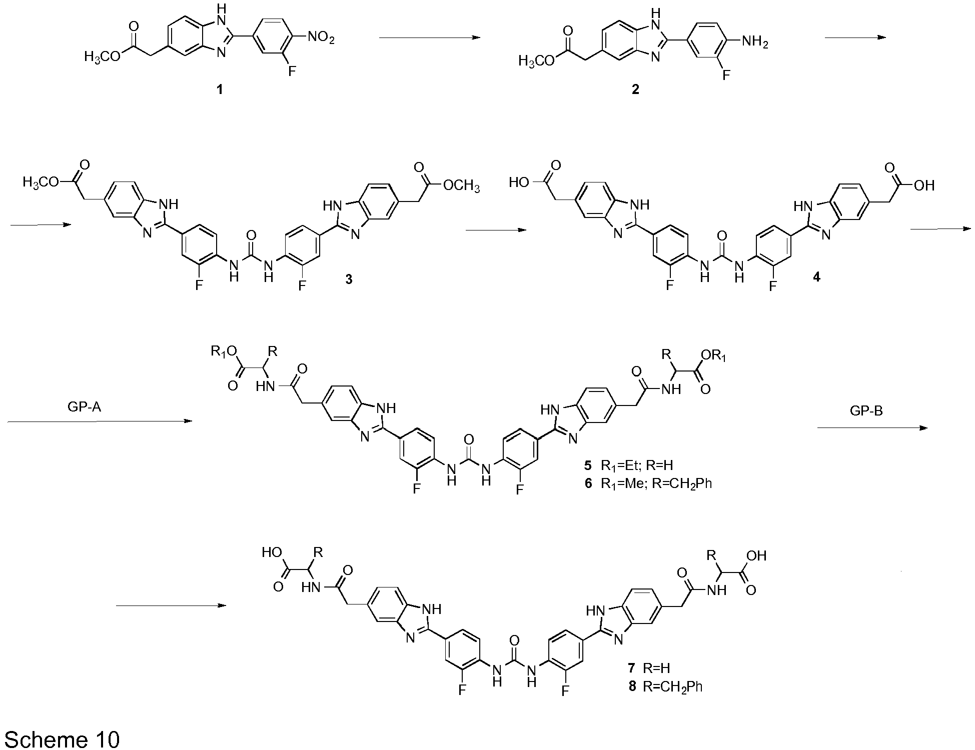

### Methyl 2-(2-(4-amino-3-fluorophenyl)-1H-benzo[d]imidazol-5-yl)acetate (2).

Diethylamine (0.2 mL, 1.9 mmol), Pd/C 10% w/w (0.078 g) and ammonium formate (1.23 g, 19 mmol) were added to a well-stirred solution of methyl 2-(2-(3-fluoro-4-nitrophenyl)-1*H-*benzo[d]imidazol-5-yl)acetate 1 (0.78 g, 1.9 mmol) (obtained according to Bahrami, K. et al, J. Org. Chem. 2008, 73(17), 6835 from the suitable starting material) in ethyl acetate (160 mL), and it was refluxed for 1h under nitrogen atmosphere. The reaction was quenched with water, cooled to room temperature, filtered on celite and washed with ethyl acetate (AcOEt). The organic layer was reduced under vacuum and the raw material was extracted with AcOEt and water. The organic phase was separated, washed with brine, dried over sodium sulphate, filtered and evaporated under vacuum to afford the pure amine. R_{f} (*n*-hexane: AcOEt 1:1): 0.15; 100% as a white solid; 247-250°C; IR *v* 3347 (NH), 3147 (NH₂), 1720 (C=O ester) cm⁻¹; ¹H NMR (dimethylsulfoxide *d₆*) δ 3.61 (s, 3H, OCH₃), 3.73-3.76 (m, 2H, CH₂ acetic), 5.67 (s, 2H, NH₂), 6.83-7.76 (m, 6H, Ar), 12.51 (bs, 1H, benzimidazole).

### Dimethyl 2,2'-(((carbonylbis(azanediyl))bis(3-fluoro-4,1-phenylene))bis(1H-benzo[d]imidazole-2,5-diyl))diacetate (3).

Triphosgene (0.23 g, 0.76 mmol) was added to a well-stirred suspension of methyl 2-(2-(4-amino-3-fluorophenyl)-1*H*-benzo[*d*]imidazol-5-yl)acetate (1.05 g, 4.58 mmol) and triethylamine (Et₃N) (0.67 mL, 4.81 mmol) in anhydrous tetrahydrofuran (THF) (49,5 mL) in an ice-cooled flask. After 10 min the ice bath was removed and the mixture was stirred at room temperature for 44 h under nitrogen atmosphere. The organic phase was reduced under vacuum, water (30 mL) was added and the solid that formed was washed with ethyl acetate, diethyl ether and petroleum ether, affording the pure product. R_{f} (ethyl acetate:methanol 20:0.1): 0.16; 100% as a white-beige solid; 220-223°C; washed with tetrahydrofuran; IR *v* 3067 (NH), 1736 (C=O ester), 1707 (C=O urea) cm^{-*1*}; ¹H NMR (dimethylsulfoxide *d₆*) δ 3.62 (s, 3H, OCH₃), 3.77-3.79 (m, 2H, CH₂ acetic), 7.10-8.45 (m, 12H, Ar), 9.43 (s, 2H, NH urea), 12.87 (bs, 2H, benzimidazole); MS: m/z (ESI) 623.1 [M-H]⁻.

### 2,2'-(((Carbonylbis(azanediyl))bis(3-fluoro-4,1-phenylene))bis(1H-benzo[d]imidazole-2,5-diyl))diacetic acid (4) - (SST0861AA1).

A solution of lithium hydroxide (0.035 g, 1.44 mmol) in distilled water (0.44 mL) was added to a solution of the ester 3 (180 mg, 0.288 mmol) in tetrahydrofuran (2.2 mL) and the reaction was stirred vigorously overnight. Upon reaction completed, the organic phase was removed under vacuum and the resulting suspension was acidified with 1 N HCl until pH 4-5 was obtained. The solid formed was collected by filtration, then washed with water and dried under IR lamp to afford pure acid. R_{f} (chloroform/ethyl acetate 1:1): 0.12; 49% as a yellow solid; 185 °C dec.; tetrahydrofuran/N,N-dimethylformamide; IR *v* 2952 (NH and OH), 1726 (C=O), 1600 (C=O) cm⁻¹; ¹H NMR (dimethylsulfoxide *d₆*) δ 3.68 (s, 4H, CH₂), 7.11-8.45 (m, 12H, Ar), 9.43 (s, 2H, NH urea), 12.46 (br s, 2H, acid), 12.86 (s, 2H, benzimidazole); 95.62%; MS: m/z (ESI) 594.8 [M-H]⁻.

### General procedure A (GP-A) to obtain amide derivatives (5) and (6).

Amino ester hydrochloride (2.4 mmol), 1-ethyl-3-(3-dimethylaminopropyl)carbodiimide (EDCI) (2.4 mmol), 4-dimethylaminopyridine (DMAP) (2.4 mmol) and N,N-diisopropylethylamine (DIPEA) (4.8 mmol) were added into a well-stirred mixture of acid 4 (1 mmol) in anhydrous N,N-dimethylformamide (DMF). The reaction was stirred at room temperature overnight under argon atmosphere. The organic phase was removed under vacuum and the crude was treated with water. The solid formed was filtered, washed with water and dried under IR lamp to afford the pure amide derivatives. For each compound amount of amino ester hydrochloride; volume of DMF; R_{f}; yield (%); melting point (°C); recrystallization solvents; IR; ¹H NMR; purity (%); and MS (ESI) are reported.

### Diethyl 2,2'-((2,2'-(2,2'-((carbonylbis(azanediyl))bis(3-fluoro-4,1-phenylene))bis(1H-benzo[d]imidazole-5,2-diyl))bis(acetyl))bis(azanediyl))diacetate (5).

Glycine ethyl ester hydrochloride (0.12 g, 0.89 mmol); anhydrous N,N-dimethylformamide 7 mL; R_{f} (ethyl acetate:methanol 1:1): 0.92; 71% as a white-grey solid; 217- 219°C; tetrahydrofuran/N,N-dimethylformamide; IR *v* 3277 (NH), 1734 (C=O ester), 1648, 1601 (C=O amide) cm⁻¹; ¹H NMR (dimethylsulfoxide *d₆*) δ 1.18 (br t, 6H, CH₃CH₂O), 3.58-3.60 (m, 4H, CH₂ acetic), 3.84 (bd, 4H, CH₂Gly), 4.10 (br q, 4H, CH₃CH₂O), 7.12-8.45 (m, 14H, Ar and NH Gly), 9.41 (s, 2H, urea), 12.82 (br s, 2H, benzimidazole); 99.99%; MS: m/z (ESI) 765.2 [M-H]⁻.

### Dimethyl 2,2'-((2,2'-(2,2'-((carbonylbis(azanediyl))bis(3-fluoro-4,1-phenylene))bis(1H-benzo[d]imidazole-5,2-diyl))bis(acetyl))bis(azanediyl))bis(3-phenylpropanoate) (6)

L-Phenylalanine methyl ester hydrochloride (0.26 g, 1.21 mmol); anhydrous N,N-dimethylformamide 9.5 mL; R_{f} (ethyl acetate:methanol 1:1): 0.75; 87% as a white-yellow solid; 210-212°C; tetrahydrofuran/N,N-dimethylformamide; IR *v* 3273 (NH), 1733 (C=O ester), 1647, 1603 (C=O amide) cm⁻¹; ¹H NMR (dimethylsulfoxide *d₆*) δ 2.90-2.96 (m, 2H, CH₂PhAla), 3.00-3.07 (m, 2H, CH₂PhAla), 3.54 (s, 4H, CH₂ acetic), 3.61 (s, 6H, CH₃O), 4.49 (m, 2H, CH PhAla), 6.98-8.45 (m, 22H, Ar), 8.55 (bd, 2H, NH amide), 9.45 (s, 2H, urea), 12.80 (br s, 1 H, benzimidazole) 99.99%; MS: m/z (ESI) 919.0 [M+H]⁺.

### General procedure B (GP-B) to obtain amino acid derivatives (7) and (8).

A solution of lithium hydroxide (5 mmol) in distilled water was added to a suspension of the appropriate ester (1 mmol) in tetrahydrofuran (fivefold the water amount) and the reaction was stirred vigorously overnight, then worked and reacted again for 24 h in order to complete the reaction. Upon reaction completed, the organic phase was removed under vacuum and the resulting suspension was acidified with 1 N HCl until pH 4-5 was obtained. The solid formed was collected by filtration, then washed with water and dried under IR lamp to afford pure acids. For each compound amount of esters; volume of solvent; R_{f}; yield (%); melting point (°C); recrystallization solvent; IR; ¹H NMR; purity (%); and MS (ESI) are reported.

### 2,2'-((2,2'-(((carbonylbis(azanediyl))bis(3-fluoro-4,1-phenylene))bis(1H-benzo[d]imidazole-2,5-diyl))bis(acetyl))bis(azanediyl))diacetic acid (7) - (SST0863AA1).

5 (0.2 g, 0.261 mmol); tetrahydrofuran 5 mL; R_{f} (ethyl acetate/methanol 1:1): 0.13; 99% as a grey solid; 190 °C dec.; tetrahydrofuran/N,N-dimethylformamide; IR *v* 3348 (OH and NH), 1727 (C=O), 1601 (C=O) cm⁻¹; ¹H NMR (dimethylsulfoxide *d₆*) δ 3.56 (s, 4H, CH₂-Ar), 3.78 (br d, 4H, CH), 7.25-8.49 (m, 14H, Ar and NH amide), 9.66 (s, 2H, NH urea), 12.6 (br s, 2H, acid); 99.84%; MS: m/z (ESI) 708.9 [M-H]⁻.

### (2S,2'S)-2,2'-((2,2'-(((Carbonylbis(azanediyl))bis(3-fluoro-4,1-phenylene))bis(1H-benzo[d]imidazole-2,5-diyl))bis(acetyl))bis(azanediyl))bis(3-phenylpropanoic acid) (8) - (SST0866AA1).

6 (0.3 g, 0.326 mmol); tetrahydrofuran 5 mL; R_{f} (ethyl acetate/methanol 1:1): 0.17; 52% as a grey solid; 198-199 °C; tetrahydrofuran/N,N-dimethylformamide; IR *v* 3270 (OH and NH), 1721 (C=O), 1637 (C=O) cm⁻¹; ¹H NMR (dimethylsulfoxide *d₆*) δ 2.89-3.09 (m, 4H, CH₂-Bz), 3.60 (s, 4H, CH₂-Ar), 4.44 (br m, 2H, CH), 7.20-8.53 (m, 24H, Ar and NH amide), 9.72 (s, 2H, NH urea), 12.6 (br s, 2H, acid); 99.56%; MS: m/z (ESI) 888.9 [M-H]⁻.

### Example 2

### Compound 9

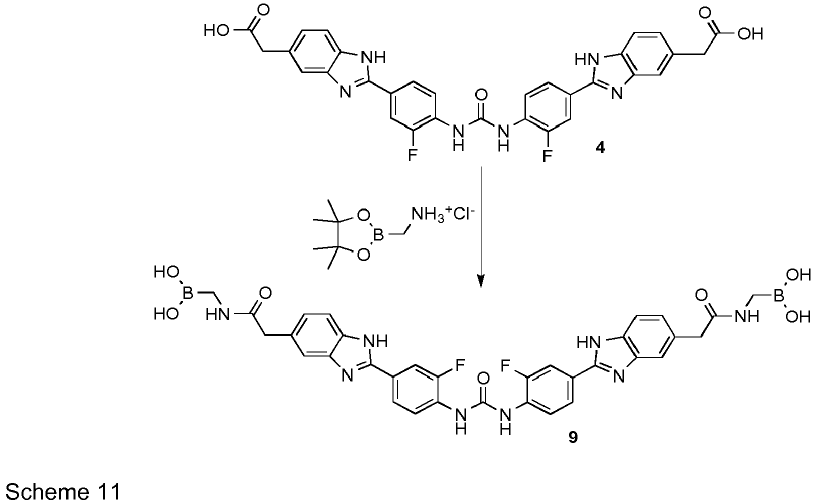

### (((2,2'-(((Carbonylbis(azanediyl))bis(3-fluoro-4,1-phenylene))bis(1H-benzo[d]imidazole-2,5-diyl))bis(acetyl))bis(azanediyl))bis(methylene))diboronic acid (9) - (SST0880AA1).

Acid derivative 4 (300 mg, 0.5 mmol), (4,4,5,5-tetramethyl-1,3,2-dioxaborolan-2-yl)methanaminium chloride (240 mg, 1.2 mmol) and O-(benzotriazol-1-yl)- N,N,N',N'-tetramethyluronium tetrafluoroborate (TBTU) (2.75 mmol) were solubilized in a mixture of anhydrous tetrahydrofuran (THF) and anhydrous N,N-dimethylformamide (DMF) (3.5 mL and 4.4 mL respectively), and the mixture was cooled to -80° C while stirring. Then a solution of i-Pr₂NEt (0.64 mL, 0.47mmol) in anhydrous THF (3 mL) was added dropwise during 2 h to the stirred reaction maintaining the temperature at -80°C. The mixture was stirred for another 1.5 h at the same temperature, and then slowly heated to room temperature. The crude was treated with distilled water, the solid formed was filtered, washed with water and dried under IR lamp to afford pure 9. R_{f}(ethyl acetate/methanol 2:1 + 0.1% CH₃COOH): 0.44; 35% as a light brown solid; 251 °C; DMF; IR *v* 3193 (OH and NH), 1652 (C=O), 1602 (C=O) cm⁻¹; ¹H NMR (DMSO *d₆*) δ 3.51 (s, 4H, CH₂-Ar), 4.50 (br d, 4H, *CH₂*-NH), 5.57 (s, 1 H, OH), 7.10 (br t, 2H, benzene H), 7.43-7.55 (m, 4H, benzimidazole C6-H and C7-H), 7.79 (s, 1 H, OH), 7.95-7.99 (m, 4H, benzene H and benzimidazole C4-H), 8.41 (br d, 2H, benzene H), 8.63 (br t, 2H, NH amide), 9.41 (s, 2H, NH urea), 12.82 (br s, 2H, benzimidazole NH). 96.38%; MS: m/z (ESI) 675.2 [M-2H₂0+H]⁺.

### Example 3

### Compound 12

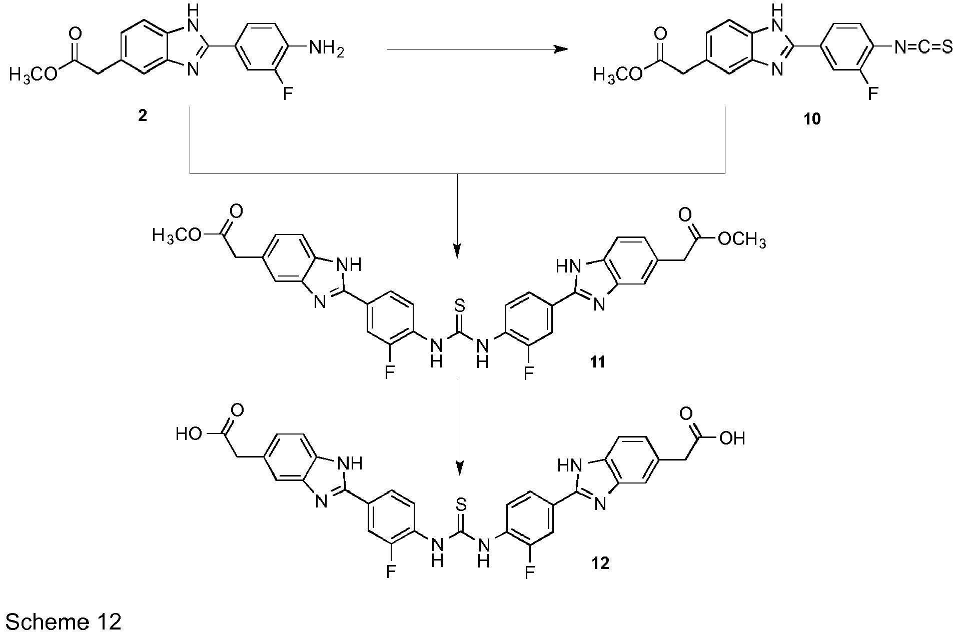

### Methyl 2-(2-(3-fluoro-4-isothiocyanatophenyl)-1H-benzo[d]imidazol-5-yl)acetate (10).

A mixture of methyl 2-(2-(4-amino-3-fluorophenyl)-1H-benzo[d]imidazol-5-yl)acetate 2 (0.5g, 1.67 mmol), thiophosgene (0.25g, 2.2 mmol, 0.16 mL) in chlorobenzene (3.5 mL) and N,N-dimethylformamide (0.013mL) was prepared at room temperature and slowly heated to reflux. Heating at reflux and stirring were continued for 4 h. At the end of this time the mixture was cooled and concentrated under reduced pressure to give 0.56 g as pure product. R_{f} (*n-*hexane/ethyl acetate 1:1): 0.51; 100% as a yellow solid; 180-181°C; IR v 2927 (NH benzimidazole), 2020 (isothiocyanate), 1739 (C=O ester) cm⁻¹; ¹H NMR (dimethylsulfoxide *d₆*) δ 3.64 (s, 3H, CH₃), 3.92 (s, 2H, CH₂), 7.34-7.43 (m, 2H, benzimidazole C4-H and benzene H), 7.66-7.82 (m, 2H, benzimidazole C6-H and C7-H), 8.20 (d, 1H, J = 8Hz, benzene H), 8.40 (d, 1 H, J = 8Hz, benzene H).

### Dimethyl 2,2'-(((thiocarbonylbis(azanediyl))bis(3-fluoro-4,1-phenylene))bis(1H-benzo[d]imidazole-2,5-diyl))diacetate (11).

A mixture of 10 (200 mg, 0.59 mmol) and 2 (166 mg, 0.56 mmol) in anhydrous dimethylsulfoxide (1.4 mL) was irradiated with microwave at 80°C for 3 min under argon atmosphere. After cooling, water was added (5.5 mL) and the precipitate that formed was filtered. The solid was washed with water and tetrahydrofuran and used for the next step without purification. R_{f} (ethyl acetate): 0.37; 30% as a dark yellow solid; 205 °C; IR *v* 3270 (NH), 2927 (NH), 1726 (C=O), 1327 (C=S) cm⁻¹; ¹H NMR (dimethylsulfoxide *d6*) δ 3.64 (s, 6H, CH₃), 3.90 (s, 4H, CH₂-Ar), 7.30-7.36 (m, 2H, benzene H), 7.65-7.72 (m, 4H, benzimidazole C6-H and C7-H), 8.06-8.23 (m, 5H, benzene H and benzimidazole C4-H), 8.54 (br t, 1 H, benzimidazole C7-H), 9.75 (s, 1 H, NH-thiourea), 10.36 (s, 1 H, SH-thiourea); 95.62%; MS: m/z (ESI) 638.8 [M-H]⁻.

### 2,2'-(((Thiocarbonylbis(azanediyl))bis(3-fluoro-4,1-phenylene))bis(1H-benzo[d]imidazole-2,5-diyl))diacetic acid (12) - (SST0870AA1).

A mixture of NaOH (31 mg, 0.78 mmol) in H₂O (5 mL) was added to a suspension of the ester 11 (100 mg, 0.156 mmol) in tetrahydrofuran (25 mL) and the mixture was stirred at room temperature overnight. The solvent was removed under vacuum and the crude was treated with 1 N HCl until pH 3-4, the solid formed was filtered, washed with water and dried under IR lamp. R_{f}(chloroform/methanol 1:1): 0.27; 90% as a grey solid; 260 °C dec.; IR *v* 2854 (OH and NH), 1710 (C=O), 1600 (C=O), 1326 (C=S) cm⁻¹; ¹H NMR (dimethylsulfoxide *d₆*) δ 3.77 (s, 4H, CH₂-Ar), 7.31 (br t, 2H, benzene H), 7.63-7.68 (m, 4H, benzimidazole C6-H and C7-H), 8.08-8.21 (m, 4H, benzene H and benzimidazole C4-H), 8.53 (br t, 2H, benzimidazole C7-H), 9.71 (s, 2H, thiourea); 99.75%; MS: m/z (ESI) 594.9 [M-H₂S+H₂0-H]⁻, 596.8 [M - H₂S + H₂0+H]⁺.

### Example 4

### Compounds 19-21

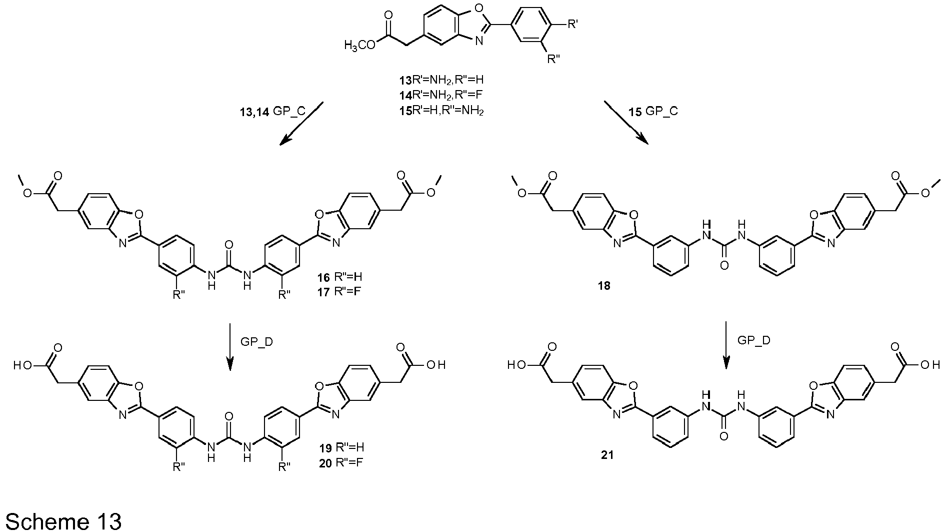

### General procedure C (GP-C) to obtain acetates (16), (17) and (18).

Triphosgene (0.18 mmol) to a stirred solution of the appropriate amine compounds (1 mmol) and trimethylamine (Et₃N) (1.05mmol) in CH₂Cl₂ in an ice-cooled flask was added. After 10 min the ice bath was removed and the mixture was stirred overnight under nitrogen atmosphere. The solvent was removed under vacuum and the crude was treated with distilled water, the solid formed was filtered, washed with water and dried under IR lamp to afford the products. For each compound amount of amine; CH₂Cl₂; chromatography eluent (if applicable); R_{f}; yield (%); melting point (°C); recrystallization solvent IR, ¹H NMR; purity (%); and MS (ESI) are reported.

### Dimethyl 2,2'-(((carbonylbis(azanediyl))bis(4,1-phenylene))bis(benzo[d]oxazole-2,5-diyl)) diacetate (16).

Methyl 2-(2-(4-aminophenyl)benzo[d]oxazol-5-yl)acetate 13 (0.90 g, 3.18 mmol); 130 mL; 30:1 chloroform/methanol; R_{f} (chloroform/methanol 10:1): 0.4; 40% as a yellow solid; 275 °C; tetrahydrofuran; IR v 3285 (NH), 1731 (C=O ester), 1638 (C=O urea) cm⁻¹; ¹H NMR (N,N-dimethylformamide d₇) δ 3.63 (s, 6H, CH₃), 3.82 (s, 4H, CH₂), 7.31 (d, 2H, benzoxazole C6-H, J = 8 Hz), 7.65-7.67 (m, 4H, benzoxazole C4-H and C7-H), 7.80 (d, 4H, benzene H, J = 8 Hz), 8.14 (d, 4H, benzene H, J = 8 Hz), 9.30 (br s, 2H, NH); MS: m/z (ESI) 590.9 [M+H]⁺.

### Dimethyl 2,2'-(((carbonylbis(azanediyl))bis(3-fluoro-4,1-phenylene))bis(benzo[d]oxazole-2, 5-diyl))diacetate (17).

Methyl 2-(2-(4-amino-3-fluorophenyl)benzo[d]oxazol-5-yl)acetate 14 (2.7 g, 9 mmol); 200 mL; 30:1 chloroform/methanol; R_{f} (chloroform/methanol 30:1): 0.18; 58% as a yellow solid; 295°C; methanol; IR v 3381 (NH), 1733 (C=O urea), 1706 (C=O ester) cm⁻¹; ¹H NMR (N,N-dimethylformamide d₇) δ 3.63 (s, 6H, CH₃), 3.83 (s, 4H, CH₂), 7.33 (d, 2H, benzoxazole C6-H, J = 8 Hz), 7.66-7.69 (m, 4H, benzoxazole C4-H and C7-H), 8.02-8.04 (m, 4H, benzene H), 8.57 (d, 2H, benzene H, J = 8 Hz), 9.60 (br s, 2H, NH); MS: m/z (ESI) 627.1 [M+H]⁺.

### Dimethyl 2,2'-(((carbonylbis(azanediyl))bis(3,1-phenylene))bis(benzo[d]oxazole-2,5-diyl)) diacetate (18).

Methyl 2-(2-(3-aminophenyl)benzo[d]oxazol-5-yl)acetate 15 (4.04 g, 14.31 mmol); 100 mL; R_{f} (chloroform,/methanol 30:1): 0.2; 100% as a white solid; 298-299°C; N,N-dimethylformamide; IR v 3280 (NH), 1732 (C=O ester), 1637 (C=O urea) cm-¹; ¹H NMR (dimethylsulfoxide d*₆*) δ 3.63 (s, 6H, CH₃), 3.84 (s, 4H, CH₂), 7.26 (d, 2H, benzoxazole C6-H, J = 8 Hz), 7.46-7.49 (m, 4H, benzoxazole C4-H and C7-H), 7.65-7.77 (m, 6H, benzene H), 8.54 (s, 2H, benzene H), 9.05 (s, 2H, NH); 99.99%; MS: m/z (ESI) 590.8 [M+H]⁺.

### General Procedure D (GP-D) to obtain acetic acids (19), (20) and (21).

A solution of lithium hydroxide (5 mmol) in distilled water was added to a suspension of the appropriate ester (1 mmol) in tetrahydrofuran (THF) (fivefold the water amount) and the reaction was stirred vigorously overnight. The organic phase was removed under vacuum and the resulting suspension was acidified with 1N HCl until pH 4-5 was obtained. The solid formed was collected by filtration, then washed with water and dried under IR lamp to afford pure acids. For each compound amount of ester; THF; R_{f}, yield (%); melting point (°C); recrystallization solvent; IR; ¹H NMR; purity (%) and MS (ESI) are reported.

### 2,2'-(((Carbonylbis(azanediyl))bis(4,1-phenylene))bis(benzo[d]oxazole-2,5-diyl))diacetic acid (19) - (SST0564AA1).

16 (0.8 g, 1.35 mmol); 17.7 mL; R_{f}(chloroform/methanol 1:1): 0.7; 62% as a white solid; 305 °C dec.; THF; IR v 3329 (OH acid), 2489 (NH), 1691 (C=O acid), 1596 (C=O urea) cm⁻¹; ¹H NMR (N,N-dimethylformamide d₇) δ 3.64 (s, 4H, CH₂), 7.24 (d, 2H, benzoxazole C6-H, J = 9 Hz), 7.54 (d, 2H, benzoxazole C7-H, J = 8 Hz), 7.85-8.07 (m, 10 H, benzene H and benzoxazole C4-H), 12.12 (br s, 2H, OH acid); 95.62%; MS: m/z (ESI) 562.8 [M+H]⁺.

### 2,2'-(((Carbonylbis(azanediyl))bis(3-fluoro-4,1-phenylene))bis(benzo[d]oxazole-2,5-diyl))diacetic acid (20) - (SST0578AA1).

17 (0.27 g, 0.43 mmol); 20 ml; R_{f} (chloroform/methanol 10:1): 0.07; 80% as a white solid; 290 °C dec. ; THF; IR v 3266 (OH acid), 2921 (NH), 1721 (C=O urea), 1694 (C=O acid) cm⁻¹; ¹H NMR (N,N-dimethylformamide d₇) δ 3.62 (s, 4H, CH₂), 7.19 (d, 2H, benzoxazole C6-H, J = 4 Hz), 7.50-7.55 (m, 4H, benzoxazole C4-H and C7-H), 7.88 (m, 4 H, benzene H, J = 8 Hz), 8.43 (d, 2 H, benzene H, J = 8 Hz), 9.48 (s, 2H, NH); 12.48 (br s, 2H, OH acid); 95.62%; MS: m/z (ESI) 596.5 [M-H]⁻.

### 2,2'-(((Carbonylbis(azanediyl))bis(3,1-phenylene))bis(benzo[d]oxazole-2,5-diyl))diacetic acid (21) - (SST0577AA1)

18 (0.15g, 0.26mmol); 75 mL; R_{f} (chloroform/methanol 1:1): 0.5; 70% as a white solid; 300°C dec.; N,N-dimethylformamide; IR v 3279 (OH acid), 2953 (NH), 1697 (C=O acid), 1694 (C=O urea) cm⁻¹; ¹H NMR (DMF d₇) δ 3.72 (s, 4H, CH₂), 7.44 (d, 2H, benzoxazole C6-H, J = 8 Hz), 7.60 (d, 2 H, benzene H, J = 8 Hz), 7.82-7.92 (m, 6H, benzoxazole C4-H andC7-H and 2H,benzene H), 8.74 (s, 2 H, benzene H), 9.80 (s, 2H, NH); 12.48 (br s, 2H, OH acid); 95.62%; MS: m/z (ESI) 563.1 [M+H]⁺.

### Example 5

### Compounds 25-28

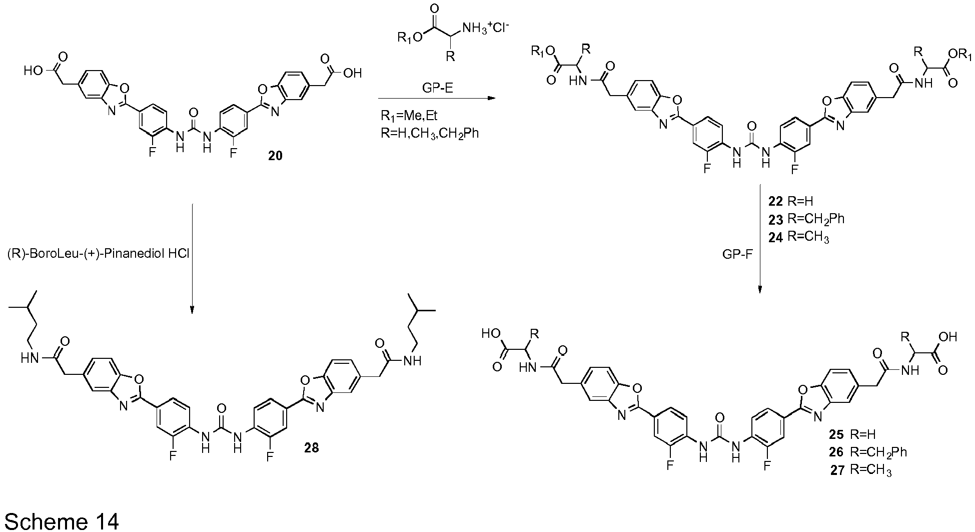

### General Procedure E (GP-E) to obtain amides (22), (23), and (24).

Amino ester hydrochloride (2.4 mmol), 1-ethyl-3-(3-dimethylaminopropyl)carbodiimide (EDCI) (2.4 mmol), 4-dimethylaminopyridine (DMAP) (2.4 mmol) and N,N-diisopropylethylamine (DIPEA) (4.8 mmol) were added into a well-stirred mixture of acid derivative (1 mmol) in anhydrous tetrahydrofuran (THF). The reaction was stirred to room temperature overnight under argon atmosphere. The organic phase was removed under vacuum and the crude was treated with water. The solid formed was filtered, washed with water and dried under IR lamp to afford the pure amide derivatives. For each compound amount of amino ester hydrochloride; dry THF; R_{f}; yield (%); melting point (°C); recrystallization solvent; IR; ¹H NMR; purity (%) and MS (ESI) are reported.

### Diethyl 2,2'-((2,2'-(2,2'-((carbonylbis(azanediyl))bis(3-fluoro-4,1-phenylene))bis(benzo[d] oxazole-5,2-diyl))bis(acetyl))bis(azanediyl))diacetate (22).

Glycine ethyl ester hydrochloride (0.11 g, 0.79 mmol); 50 mL; R_{f}(chloroform/methanol 40:1): 0.66; 72% as an orange solid; 174-176°C; washed with tetrahydrofuran; IR: *v* 3289 (NH), 1728 (C=O ester), 1649, 1600 (C=O amide) cm⁻¹; ¹H NMR (N,N-dimethylformamide *d₇*) 51.39 (t, 6H, CH₃CH₂O, J=8 Hz), 3.95 (s, 4H, CH₂ acetic), 4.17 (d, 4H, CH₂ Gly, J=8 Hz), 4.31 (q, 4H, CH₃CH₂O, J=8 Hz), 7.60-8.86 (m, 14H, Ar and NH Gly), 9.88 (s, 2H, urea) 99.99%; MS: m/z (ESI) 766.7 [M-H]⁻.

### Dimethyl 2,2'-((2,2'-(2,2'-((carbonylbis(azanediyl))bis(3-fluoro-4,1-phenylene))bis(benzo[d]oxazole-5,2-diyl))bis(acetyl))bis(azanediyl))bis(3-phenylpropanoate) (23).

L-Phenylalanine methyl ester hydrochloride (0.17 g, 0.79 mmol); 50 mL; R_{f} (chloroform/methanol 40:1): 0.87; 64% as an orange solid; 182-185°C; washed with tetrahydrofuran; IR *v* 3267 (NH), 1733 (C=O ester), 1648, 1599 (C=O amide) cm⁻¹; ¹H NMR (dimethylsulfoxide *d₆*) δ 2.89-2.93 (m, 2H, CH₂PhAla), 3.00-3.08 (m, 2H, CH₂PhAla), 3.57 (s, 4H, CH₂ acetic), 3.61(s, 6H, OCH₃), 4.46-4.52 (m, 2H, CH PhAla), 7.17-7.24 (m, 12H, benzene and benzoxazole C6-H), 7.59 (s, 2H, benzoxazole C4-H), 7.65 (d, 2H, benzoxazole C7-H, J = 8 Hz), 8-8.05 (m, 4H, benzene H), 8.53(t, 2H, benzene H, J = 8 Hz), 8.63 (d, 2H, NH-CO, J = 8 Hz), 9.62 (s, 2H, NH urea); 99.99%; MS: m/z (ESI) 918.9 [M-H]⁻.

### Diethyl 2,2'-((2,2'-(2,2'-((carbonylbis(azanediyl))bis(3-fluoro-4,1-phenylene))bis(benzo[d]oxazole-5,2-diyl))bis(acetyl))bis(azanediyl))dipropanoate (24).

L-Alanine ethyl ester hydrochloride (0.2 g, 1.28 mmol); 75 mL; R_{f}(chloroform/methanol 4:1): 0.77; 85% as a white solid; 235-238°C; washed with tetrahydrofuran; IR *v* 3280 (NH), 1732 (C=O ester), 1646, 1601 (C=O amide) cm⁻¹; ¹H NMR (dimethylsulfoxide *d₆*) δ 1.15 (t, 6H, CH₃CH₂O, J = 8 Hz), 1.30 (d, 6H, CH₃PhAla, J = 4 Hz), 3.61 (s, 4H, CH₂ acetic), 4.04-4.09 (m, 4 H, CH₃CH₂O), 4.22-4.26 (m, 2 H, CH Ala), 7.31 (d, 2H, benzoxazole C6-H, J = 8 Hz), 7.60-7.70 (m, 4H, benzoxazole C7-H and C4-H), 7.90-8.05 (m, 4H, benzene H), 8.52 (t, 2H, benzene H, J = 8 Hz) 8.60 (d, 2H, NHCO, J = 8 Hz), 9.61 (s, 2H, NH urea); 99.99%; MS: m/z (ESI) 794.5 [M-H]⁻.

### General Procedure F (GP-F) to obtain acylamino acid derivatives (25), (26) and (27).

A solution of lithium hydroxide (5 mmol) in distilled water was added to a suspension of the appropriate ester (1 mmol) in tetrahydrofuran (fivefold the water amount) and the reaction was stirred vigorously for the required time. The organic phase was removed under vacuum and the resulting suspension was acidified with 1 N HCl until pH 4-5 was obtained. The solid formed was collected by filtration, then washed with water and dried under IR lamp to afford pure acids. For each compound amount of ester; tetrahydrofuran; reaction time; R_{f}; yield (%); melting point (°C); recrystallization solvent; IR; ¹H NMR; purity (%) and MS (ESI) are reported.

### 2,2'-((2,2'-(((Carbonylbis(azanediyl))bis(3-fluoro-4,1-phenylene))bis(benzo[d]oxazole-2,5-diyl))bis(acetyl))bis(azanediyl))diacetic acid (25) - (SST0795AA1).

22 (0.10 g , 0.130 mmol); 50 mL; overnight; R_{f} (chloroform/methanol 3:7): 0.54; 65% as a yellow solid; 269 °C dec. ; IR *v* 3282 (NH), 3077 (OH), 1726 (C=O), 1727 (C=O), 1650 (C=O), 1600 (C=O) cm⁻¹; ¹H NMR (dimethylsulfoxide *d₆*) δ 3.62 (s, 4H, CH₂), 3.77 (d, 4H, J = 4 Hz, *CH₂*-NH), 7.32 (d, 2H, J = 8 Hz, benzoxazole C6-H), 7.68-7.70 (m, 4H, benzoxazole C4-H and C7-H), 8.00-8.04 (m, 4H, benzene H), 8.44 (br t, 2H, *NH*-CH₂), 8.52 (t, 2H, J = 8 Hz, benzene H), 9.6 (br s, 2H, NH-C=O). 95.62%; MS: m/z (ESI) 711.0 [M-H]⁻.

### (2S,2'S)-2,2'-((2,2'-(((Carbonylbis(azanediyl))bis(3-fluoro-4,1-phenylene))bis(benzo[d]oxazole-2,5-diyl))bis(acetyl))bis(azanediyl))bis(3-phenylpropanoic acid) (26) - (SST0797AA1).

23 (0.17 g, 0.184 mmol); 87.5 ml; 3.5 h; R_{f}(chloroform/methanol 3:7): 0.55; 95% as a yellow solid; 210 °C dec.; IR *v* 3268 (OH and NH), 2973 (NH), 1716 (C=O), 1649 (C=O) cm⁻¹; ¹H NMR (dimethylsulfoxide *d₆*) δ 2.86-3.10 (m, 4H, CH₂-Bz), 3.56 (s, 4H, CH₂-Ar), 4.42-4.47 (m, 2H, CH), 7.17-7.24 (m, 12H, benzene H and benzoxazole C6-H), 7.59 (s, 2H, benzoxazole C4-H), 7.64 (d, 2H, benzoxazole C7-H, J = 8 Hz), 8-8.05 (m, 4H, benzene H), 8,47 (d, 2H, NH-CO, J = 8 Hz), 8.53(t, 2H, benzene H, J = 8 Hz), 9.63 (s, 2H, NH urea), 13.5 (br s, 2H, acid); 99.74%; MS: m/z (ESI) 890.9 [M-H]⁻.

### (2S,2'S)-2,2'-((2,2'-(((Carbonylbis(azanediyl))bis(3-fluoro-4,1-phenylene))bis(benzo[d]oxazole-2,5-diyl))bis(acetyl))bis(azanediyl))dipropionic acid (27) - (SST0796AA1).

24 (0.33 g, 0.42 mmol); 167.5 ml; 3.5 h; R_{f}(chloroform/methanol 3:7): 0.51; 89% as a white-yellow solid; 232 °C dec.; IR *v* 3267 (OH and NH), 2956 (NH), 1712 (C=O), 1608 (C=O) cm⁻¹; ¹H NMR (dimethylsulfoxide *d₆*) δ 1.28 (br d, 6H, CH₃), 3.60 (s, 4H, CH₂-Ar), 4.18 (br m, 2H, CH), 7.31 (br d, 2H, benzoxazole C6-H), 7.60-7.70 (m, 4H, benzoxazole C7-H and C4-H), 7.90-8.05 (m, 4H, benzene H), 8.52 (t, 2H, benzene H, J = 8 Hz) 8.60 (d, 2H, NH-CO, J = 8 Hz), 9.61 (s, 2H, NH urea), 12.7 (br s, 2H, acid); 99.88%; cc, 738.8 [M-H]⁻.

### 2,2'-(((Carbonylbis(azanediyl))bis(3-fluoro-4,1-phenylene))bis(benzo[d]oxazole-2,5-diyl))bis(N-isopentylacetamide) (28) - (SST0862AA1).

(R)-BoroLeu-(+)-pinanediol HCl (0.39 g, 1.28 mmol), EDCI (0.25 g, 1.28 mmol), DMAP (0.16 g, 1.28 mmol) and DIPEA (0.33 g, 2.56 mmol, 0.45 ml) were added to a well stirred solution of the acetic acid 20 (0.30 g, 0.533 mmol) in tetrahydrofuran dry (75 ml). The mixture was stirred at room temperature overnight under argon atmosphere. The organic phase was removed under vacuum and the resulting suspension was treated with water. The solid formed was collected by filtration, then washed with water and dried under IR lamp to afford pure derivative as a white solid. R_{f} (chloroform/methanol 4:1): 0.63; 87%; carbonizes at 248 °C; IR *v* 3284 (NH), 2955 (NH), 1674 (C=O), 1608 (C=O) cm⁻¹; ¹H NMR (dimethylsulfoxide *d₆*) δ 0.86 (d, 12H, J=8 Hz, CH₃-ⁱPr), 1.30 (q, 4H, *J* = 8 Hz, CH₂-ⁱPr), 1.54-1.59 (m, 2H, CH-ⁱPr), 3.06 (q, 4H, *J* = 8 Hz, αCH₂N), 3.52 (s, 4H, CH₂-Ar), 7.30 (d, 2H, *J* = 8 Hz, benzoxazole C6-H), 7.64 (s, 2H, benzoxazole C4-H), 7.69 (d, 2H, *J* = 8 Hz, benzoxazole C7-H), 7.99-8.07 (m, 5H, benzene H and NH amide), 8.52 (t, 2H, *J*= 8 Hz, benzene H), 9.60 (s, 2H, NH urea); 99.02%; MS: m/z (MALDI) 367.3 [M-2H]²⁻/2.

### Example 6

### Compounds 30-31

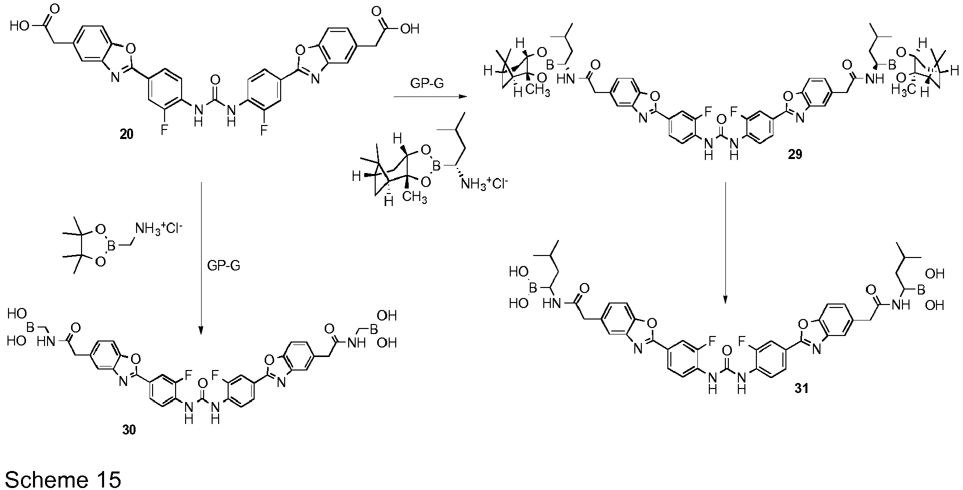

### General procedure G (GP-G) to obtain boronic derivatives (29) and (30).

The appropriate ammonium salt (2.5 mmol), 20 (1 mmol), and O-(benzotriazol-1-yl)-N,N,N',N'-tetramethyluronium tetrafluoroborate (TBTU) (2.75 mmol) were solubilized in anhydrous N,N-dimethylformamide, and the mixture was cooled to -80° C while stirring. After that a solution of i-Pr₂NEt (7.25mmol) in anhydrous N,N-dimethylformamide (4 mL) was added dropwise during 2 h to a stirred reaction mixture maintaining the temperature at -80°C. The mixture was stirred for another 1.5 h, and then slowly heated to room temperature. The solvent was removed under vacuum and the crude was treated with distilled water, the solid formed was filtered, washed with water and dried under IR lamp to afford the products. For each compound amount of acid and ammonium salt; N,N-dimethylformamide; Rf; yield (%); melting point (°C); IR; ¹H NMR; purity (%) and MS (ESI) are reported.

### 2-(2-(3-fluoro-4-(3-(2-fluoro-4-(5-(2-((3-methyl-1-((3aR,4R,6R,7aS)-5,5,7a-trimethylhexahydro-4,6-methanobenzo[d][1,3,2]dioxaborol-2-yl)butyl)amino)-2-oxoethyl)benzo[d]oxazol-2-yl)phenyl)ureido)phenyl)benzo[d]oxazol-5-yl)-N-(3-methyl-1-((3aS,4S,6S,7aR)-5,5,7a-trimethylhexahydro-4,6-methanobenzo[d][1,3,2]dioxaborol-2-yl)butyl)acetamide (29).

20 (0.3g, 0.5 mmol) and (R)-boroLeu-(+)-pinanediol HCl (0.38 g, 1.2 mmol); 2 mL; R_{f} (chloroform/methanol 4:1): 0.42; 96.2% as a yellow solid; 189 °C; washed with diethyl ether; IR *v* 3274 (OH and NH), 2922 (NH), 1726 (C=O), 1601 (C=O) cm⁻¹; ¹H NMR (dimethylsulfoxide *d₆*) δ 0.80-2.33 (m, 50H, αCH-B, 10CH₃, 6CH₂, 6CH), 3.75 (s, 4H, CH₂Ar), 4.00 (d, 2H, J=8 Hz, CHpinane-O), 7.30 (d, 2H, *J* = 8 Hz, benzoxazole C6-H), 7.68 (s, 2H, C4-H), 7.74 (d, 2H, *J* = 8 Hz, benzoxazole C7-H), 7.99-8.05 (m, 4H, benzene H), 8.52 (t, 2H, *J* = 8 Hz, benzene H), 9.40 (s, 2H, NH amide), 9.60 (s, 2H, NH urea); 98.40%; MS: m/z (ESI) 1090.7 [M-H]⁻.

### (((2,2'-(((Carbonylbis(azanediyl))bis(3-fluoro-4,1-phenylene))bis(benzo[d]oxazole-2,5-diyl))bis(acetyl))bis(azanediyl))bis(methylene))diboronic acid (30).

20 (0.3 g, 0.5 mmol) and (4,4,5,5-tetramethyl-1,3,2-dioxaborolan-2-yl)methanaminium chloride (0.24 g, 1.2 mmol); 2 mL; R_{f} (methanol + 0.05% TFA): 0.12; 30% as a white solid; 237 °C dec.; N,N-dimethylformamide; IR *v* 3356 (OH and NH), 2962 (NH), 1718 (C=O), 1604 (C=O) cm⁻¹; ¹H NMR (dimethylsulfoxide *d₆*) δ 2.27 (d, 4H, J=4 Hz, αCH₂-B), 3.56 (s, 4H, CH₂Ar), 6.90 (bt, 2H, NH amide), 7.32-8.54 (m, 12H, Ar), 9.60 (s, 2H, NH urea); 99.64%; MS: m/z (ESI) 710.8 [M-H]⁻.

### (1R,1'R)-(((2,2'-(((Carbonylbis(azanediyl))bis(3-fluoro-4,1-phenylene))bis(benzo[d]oxazole-2,5-diyl))bis(acetyl))bis(azanediyl))bis(3-methylbutane-1,1-diyl))diboronic acid (31) -(SST0878AA1).

To a stirred solution of the boronate ester 29 (0.20 g, 0.183 mmol) in tetrahydrofuran was added aqueous ammonium acetate (7.96 ml, 0.1 N) and sodium periodate (0.23 g, 1.07 mmol). The mixture was stirred vigorously at room temperature for 47 h, then the tetrahydrofuran was removed under vacuum and the resulting solid was collected by filtration, washed with water and dried under IR lamp, then washed with dichloromethane and reacted again in the same conditions described above for 24 h. The tetrahydrofuran was removed under vacuum and the resulting solid was filtered, washed with water and dried under IR lamp prior to be washed with dichloromethane. R_{f} (chloroform/methanol 1:1): 0.67; 60% as a grey solid; 226 °C; tetrahydrofuran/N,N-dimethylformamide; IR *v* 3271 (OH and NH), 2956 (NH), 1603 (C=O) cm⁻¹; ¹H NMR (dimethylsulfoxide *d₆*) δ 0.84 (s, 12H, CH₃), 1.31-1.61 (m, 6H, 2CH₂-ⁱPr and 2CH(CH₃)₂), 3.52 (s, 4H, CH₂Ar), 5.22 (s, 2H, αCH-B), 5.56 (s, 4H, OH), 7.30 (d, 2H, *J* = 8 Hz, benzoxazole C6-H), 7.66-87.98 (m, 4H, benzoxazole C4-H and C7-H), 7.97-8.02 (m, 4H, benzene H), 8.40 (d, 2H, *J* = 8 Hz, NH amide), 8.51 (t, 2H, *J* = 8 Hz, benzene H), 9.58 (s, 2H, NH urea); 98.40%; MS: m/z (ESI) 862.7 [M-H]⁻.

### Example 7

### Compound 33

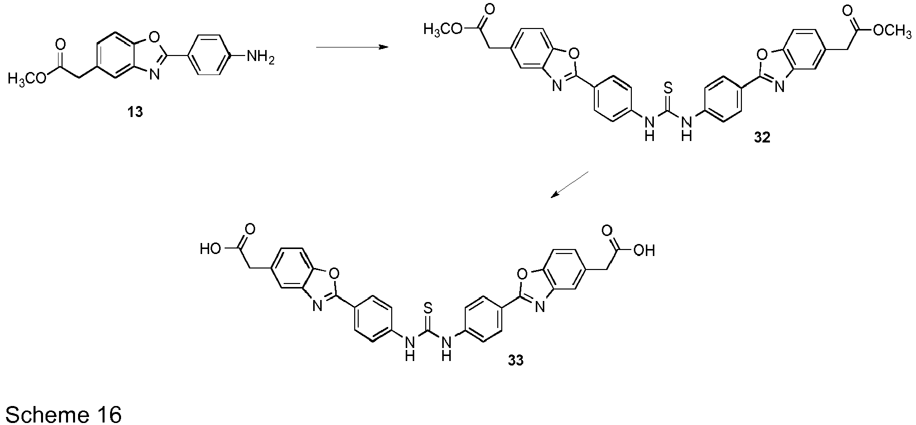

### Dimethyl 2,2'-(((thiocarbonylbis(azanediyl))bis(4,1-phenylene))bis(benzo[d]oxazole-2,5-diyl))diacetate (32).

To a stirred solution of 13 (0.61 g, 2.2mmol) in dichloromethane (100mL) was added N,N'-thiocarbonyldiimidazole (0.18g, 1.03mmol), the mixture was stirred at 40°C for 12h. The solvent was removed under vacuum and the crude was treated with distilled water, the solid formed was filtered, washed with water and dried under IR lamp. Purification of crude product was performed by chromatography (chloroform/methanol 30:1 as eluent) to give 0.385 g as pure product. R_{f} (chloroform/methanol 10:1): 0.55; 60% as a yellow solid; 195-196 °C; washed with diethyl ether ; IR v 3284 (NH), 1731 (C=O ester) cm⁻¹; ¹H NMR (N,N-dimethylformamide d₇) δ 3.80 (s, 6H, CH₃), 3.99 (s, 4H, CH₂), 7.48-7.51 (d, 2H, benzoxazole C6-H), 7.82-7.86 (m, 4H, benzoxazole C4-H and C7-H), 8.06-8.08(d, 4H, benzene H), 8.33-8.35 (d, 4H, benzene H), 10.60 (s, 2H, NH); MS: m/z (ESI) 606.8 [M+H]⁺.

### Synthesis of 2,2'-(((thiocarbonylbis(azanediyl))bis(4,1-phenylene))bis(benzo[d]oxazole-2,5-diyl))diacetic acid (33) - (SST0587AA1)

A mixture of NaOH (5 mmol) and 32 (0.84 g, 1.38 mmol) in tetrahydrofuran (50 mL) and H₂O (20 mL) was stirred at room temperature overnight. The solvent was removed under vacuum and the crude was treated with 1N HCl until pH 3-4, the solid formed was filtered, washed with water and dried under IR lamp. to give pure product (0.43g).; R_{f} (chloroform/methanol 10:1): 0.10; 44% as a brow solid; Carbonizes 300°C; washed with diethyl ether; IR v 2953 (OH acid), 2608 (NH), 1690 (C=O acid) cm⁻¹; ¹H NMR (N,N-dimethylformamide d₇) δ 3.76(s, 4H, CH₂), 7.32-7.34 (d, 2H, benzoxazole C6-H), 7.65-7.69 (m, 4H, benzoxazole C7-H and benzoxazole C4-H), 8.01-8.03 (d, 4 H, benzene H), 8.16-8.18 (d, 4H, benzene H),11.21 (br s, 2H, NH), 12.55 (br s, 2H, OH acid); MS: m/z (ESI) 576.5 [M-H]⁻.

### Example 8

### Compound 38

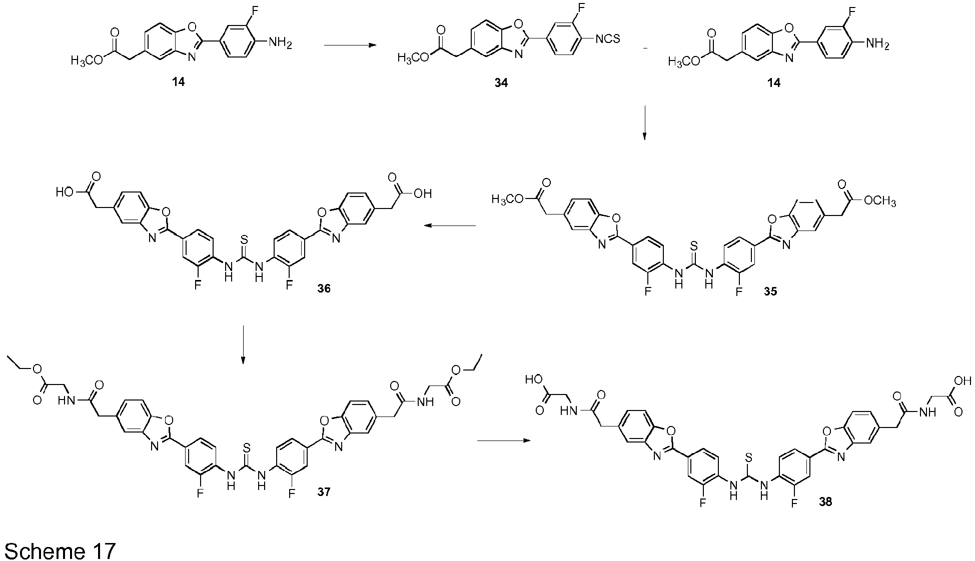

### Synthesis of methyl 2-(2-(3-fluoro-4-isothiocyanatophenyl)benzo[d]oxazol-5-yl)acetate (34).

A mixture of methyl 2-(2-(4-amino-3-fluorophenyl)benzo[d]oxazol-5-yl)acetate 14 (0.5 g, 1.66 mmol), thiophosgene (0.25 g, 2.2 mmol, 0.16 mL) in chlorobenzene (3.5 mL) and N,N-dimethylformamide (0.013 mL) was prepared at room temperature and slowly heated to reflux. Heating at reflux and stirring were continued for 2 h. at the end of this time the mixture was cooled and concentrated under reduced pressure to give 0.57 g as pure product. R_{f} (n-hexane/ethyl acetate 1:1): 0.92; 100% as an orange solid; 175-176°C; IR v 2021 (Isothiocyanate), 1732 (C=O ester) cm⁻¹; ¹H NMR (dimethylsulfoxide *d₆*) δ 3.55 (s, 3H, CH₃), 3.76 (s, 2H, CH₂), 7.27-7.29 (d, 1 H, benzoxazole C6-H), 7.59-7.66 (m, 3H, benzoxazole C4-H, C7-H and benzene H), 7.94-7.96 (d, 1 H, benzene H), 8.03-8.05 (d, 1 H, benzene H), 9.30 (br s, 2H, NH); MS: m/z (ESI) 340.1 [M+H]⁺.

### Dimethyl 2,2'-(((thiocarbonylbis(azanediyl))bis(3-fluoro-4,1-phenylene))bis(benzo[d] oxazole-2,5-diyl))diacetate (35).

A mixture of methyl 2-(2-(4-amino-3-fluorophenyl)benzo[d]oxazol-5-yl)acetate 14 (1 g, 3.33 mmol) and methyl 2-(2-(3-fluoro-4-isothiocyanatophenyl)benzo[d]oxazol-5-yl)acetate 34 (1.20 g, 3.5 mmol) in dry dimethylsulfoxide (1 mL) was irradiated with microwave at 80°C for 3 min under argon atmosphere. After cooling, water was added (5 mL) and the precipitate that formed was filtered. The solid was washed with water, dissolved in tetrahydrofuran and precipitate with n-hexane to give 1.46 g as pure product. R_{f} (chloroform/methanol 30:1): 0.23; 70% as a yellow solid; 169-170°C; washed with n-hexane; IR v 3117 (NH), 1743 (C=O ester) 1329 (C=S) cm⁻¹; ¹H NMR (dimethylsulfoxide d*₆*) δ 3.55 (s, 6H, CH₃), 3.77 (s, 4H, CH₂), 7.26-7.28 (d, 2H, benzoxazole C6-H), 7.65-7.68 (m, 4H, benzoxazole C4-H and C7-H), 7.94-7.97(d, 4H, benzene H), 8.02-8.06 (t, 2H, benzene H), 10.15 (br s, 2H, NH); MS: m/z (ESI) 643.1 [M+H]⁺.

### 2,2'-(((Thiocarbonylbis(azanediyl))bis(3-fluoro-4,1-phenylene))bis(benzo[d]oxazole-2,5-diyl))diacetic acid (36) - (SST0626AA1)

A mixture of sodium hydroxide (5 mmol) and 35 (0.13 g, 0.20 mmol) in tetrahydrofuran (50 mL) and H₂O (20 mL) was stirred at room temperature overnight. The solvent was removed under vacuum and the crude was treated with 1N HCl until pH 3-4, the solid formed was filtered, washed with water and dried under IR lamp. to give pure product (0.12 g). R_{f} (chloroform/methanol 1:1): 0.4; 100% as a yellow solid; 330°C dec.; washed with diethyl ether; IR v 3113 (OH acid), 2620 (NH), 1716 (C=O acid), 1327 (C=S) cm⁻¹; ¹H NMR (N,N-dimethylformamide d₇) δ 3.77(s, 4H, CH₂), 7.36-7.38 (d, 2H, benzoxazole C6-H), 7.68-7.72 (m, 4H, benzoxazole C7-H and benzoxazole C4-H), 7.99-8.05 (m, 4 H, benzene H), 8.23-8.27 (t, 2H, benzene H),9.68 (br s, 2H, NH), 12.60 (br s, 2H, OH acid); MS: m/z (ESI) 612.6 [M-H]⁻.

### Diethyl 2,2'-((2,2'-(((thiocarbonylbis(azanediyl))bis(3-fluoro-4,1-phenylene))bis(benzo[d]oxazole-2,5-diyl))bis(acetyl))bis(azanediyl))diacetate (37).

Glycine ethyl ester hydrochloride (0.11 g, 0.78 mmol), 1-ethyl-3-(3-dimethylaminopropyl)carbodiimide (EDCI) (0.12 g, 0.78 mmol), 4-dimethylaminopyridine (DMAP) (0.09 g, 0.78 mmol) and N,N-diisopropylethylamine (DIPEA) (0.20 g, 1.56 mmol, 0.27 mL) were added into a well-stirred mixture of 36 (0.2 g, 0.32 mmol) in anhydrous tetrahydrofuran (50 mL). The reaction was stirred to room temperature overnight under argon atmosphere. The organic phase was removed under vacuum and the crude was treated with water. The solid formed was filtered, washed with water and dried under IR lamp to afford the pure derivative as a yellow solid (0.2 g, 80%). R_{f} (chloroform:methanol 40:1) 0.8; 186-187°C; IR v 2625 (NH), 1766 (C=O ester ), 1723 (C=O amide), 1330 (C=S) cm⁻¹; ¹H NMR (N,N-dimethylformamide *d₇*) δ1.21 (t, 6H, CH₃CH₂O, J=8 Hz), 3.75-3.79 (m, 4H, CH₂NH), 3.97-4.01 (m, 4H, CH₂ Gly), 4.12 (q, 4H, CH₃CH₂O, J=8 Hz), 6.83-8.69 (m, 14H, Ar and NH Gly), 9.85 (br s, 2H, tiourea); MS: m/z (ESI) 769.3 [M - H₂S + H₂O+H]⁺.

### 2,2'-((2,2'-(((Thiocarbonylbis(azanediyl))bis(3-fluoro-4,1-phenylene))bis(benzo[d]oxazole-2,5-diyl))bis(acetyl))bis(azanediyl))diacetic acid (38). (SST0872AA1)

A mixture of sodium hydroxide (5 mmol) and 37 (0.150 g, 0.19 mmol) in tetrahydrofuran (20 mL) and H₂O (4 mL) was stirred at room temperature overnight. The solvent was removed under vacuum and the crude was treated with 1N HCl until pH 3-4, the solid formed was filtered, washed with water and dried under IR lamp to give pure product as a yellow solid (0.09 g, 70%). R_{f}(chloroform:methanol 1:1) 0.23; 200°C dec.; IR v 3277 (OH and NH), 1719 (C=O acid), 1644 (C=O amide), 1329 (C=S) cm⁻¹; ¹H NMR (DMSO *d₆*) δ 3.60-3.63 (m, 4H, CH₂NH), 3.76-3.79 (m, 4H, CH₂ Gly), 7.28-8.54 (m, 14H, Ar and NH Gly), 9.61 (br s, 2H, tiourea) 98.21 %; MS: m/z (ESI) 711.3 [M - H₂S + H₂0-H]⁻.

### Example 9

### Compound 40

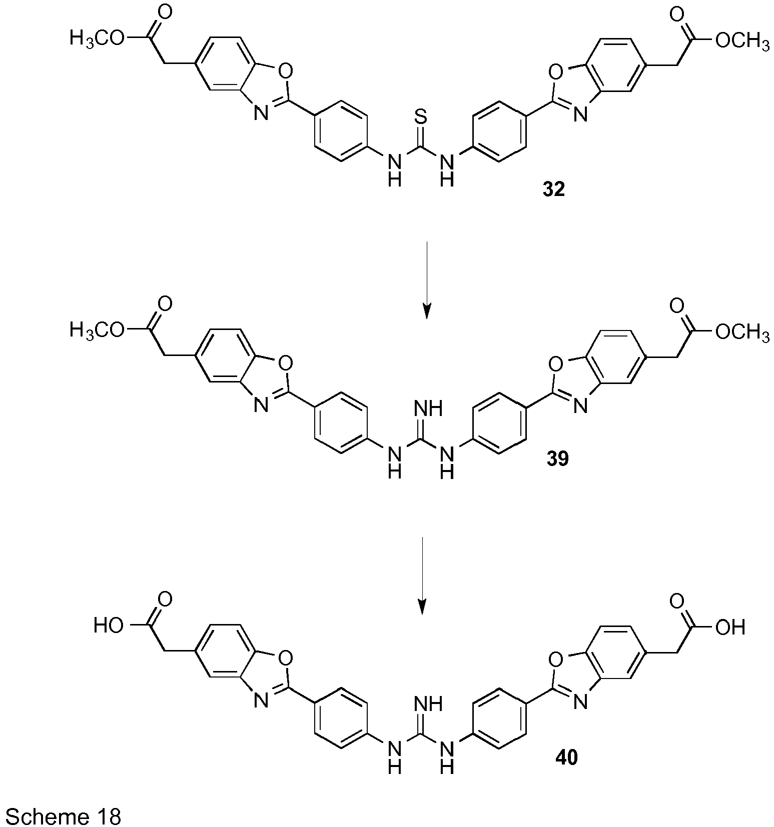

### Dimethyl 2,2'-((((iminomethylene)bis(azanediyl))bis(4,1-phenylene))bis(benzo[d]oxazole-2,5-diyl))diacetate (39)

To a stirred suspension of 32 (0.10 g, 0.165 mmol) in acetonitrile (2 mL) were added hexamethyldisilazane (0.027 g, 0.165 mmol, 0.034 mL) and dimethylaminopropyl)carbodiimide (EDCI) (0.063 g, 0.33 mmol) in an ice-bath and the mixture was stirred at room temperature for 5 days. The solvent was removed under vacuum and the crude was purified by column chromatography (aluminum oxide (Al₂O₃), 30:1 chloroform:methanol as eluent) to give the product as a yellow solid (0.07 g, 70%). R_{f} (chloroform/methanol 30:1): 0.4; 145-146 °C; washed with diethyl ether; IR v 3286 (NH), 2954 (NH), 1731 (C=O ester) cm⁻¹; ¹H NMR (dimethylsulfoxide d*₆*) δ 3.47 (s, 6H, CH₃), 3.49 (s, 4H, CH₂), 5.66 (br d, 2H, NH guanidine), 7.19 (d, 2H, J=8 Hz, C7-H benzoxazole), 7.57 (s, 2H, C4-H benzoxazole), 7.60 (d, 2H, J=8 Hz, C6-H benzoxazole), 7.63 (d, 4H, J=8 Hz, benzene H), 8.04 (d, 4H, benzene H), 9.21 (s, 2H, NH); MS: m/z (ESI) 590.4 [M+H]⁺.

### 2,2'-((((Iminomethylene)bis(azanediyl))bis(4,1-phenylene))bis(benzo[d]oxazole-2,5-diyl))diacetic acid (40) (SST0665AA1)

A solution of lithium hydroxide (0.122 g, 5.0 mmol) in distilled water (0.25 mL) was added to a solution of 39 (0.3 g, 0.51 mmol) in dimethylsulfoxide (fivefold the water amount) and the reaction was stirred vigorously for 3 days. The crude was treated with 2N HCl until pH 2-3, the solid formed was filtered, washed with water and dried under IR lamp. to give pure product as a yellow solid (0.2 g, 70%). R_{f} (chloroform/methanol 10:1): 0.71; 270°C dec.; washed with diethyl ether; IR v 3065 (OH acid), 1667 (C=O acid) cm⁻¹; ¹H NMR (dimethylsulfoxide d*₆*) δ 3.61 (s, 4H, CH₂), 5.73 (br d, 2H, NH guanidine), 7.18-7.55 (m, 10H, benzene H and benzoxazole C4-H, C6-H and C7-H), 8.00 (br d, 4H, benzene H), 9.54 (br s, 2H, NH), 11.84 (br s, 2H, OH acid); 100.00%; MS: m/z (MALDI) 562.4 [M+H]⁺.

### Example 10

### In vitro screening for heparanase activity

All compounds were tested in a simple, accurate and robust microplate assay based on the cleavage of the synthetic heparin fragment, the pentasaccharide Fondaparinux (AGA*IA). This assay was originally developed by Hammond and coworkers [Hammond E, Li CP, Ferro V. Development of a colorimetric assay for heparanase activity suitable for kinetic analysis and inhibitor screening. Anal. Biochem. 396(1), 112-116 (2010)], where Fondaparinux corresponds to the methyl glycoside of the antithrombin III (ATIII)-activating pentasaccharide sequence of heparin. Fondaparinux is cleaved by heparanase to generate a reducing disaccharide, which can be quantitatively measured via a colorimetric or a fluorescence assay, the last by the fluorescent sensor polymer-H [Schiemann S, Lühn S, Alban S. Development of both colorimetric and fluorescence heparinase activity assays using fondaparinux as substrate. Anal. Biochem. 427(1), 82-89 (2012)].

### Materials and methods

To determine the activity of the newly designed heparanase inhibitors an homogenous assay based on the cleavage of the synthetic heparin oligosaccharide fondaparinux has been employed (Hammond E. et al. Anal. Biochem. 2010, 396, 112). The assay measures the appearance of the disaccharide product of heparanase-catalyzed fondaparinux cleavage colorimetrically using the tetrazolium salt WST-1. Assay was essentially performed as described with minor modifications. Briefly, NUNC ELISA 96-well plates were pre-treated with a solution of 4% BSA (bovin serum albumin) in phosphate-buffered saline containing 0.05% Tween 20 (PBST), for 2 h at 37°C, then washed three times with PBST, dried by tapping on paper towel and stored at -20°C. Before use, once again, the plates were washed with PBST.

Assay was carried out with 100 µl/well of assay solution containing 40 mM sodium acetate buffer (pH 5.0), 100 µM fondaparinux, 2.5 nM heparanase and serial dilutions of test compounds (tested in triplicate). Plates were sealed with adhesive tape and incubated, in the dark, for 3 h at 37°C, followed by development with 1.69 mM of the tetrazolium salt WST-1 solution in 0.1 M NaOH, for 1 h at 60°C. Then, the absorbance at 560 nm was measured through a microplate reader (Victor 3, Perkin Elmer).

IC50 value for each compound, versus heparanase, was calculated by GraphPad software and results of selected compounds are summarized in the Table 1 below. Finally, the measurements were corrected by subtracting both the reagent background and the inner absorbance value of test compound.

### Results

**Table 1. IC₅₀ value in AGA*IA assay, versus heparanase inhibition.**

| Compound | Code lab | AGA*IA assay IC₅₀ (nM) |
|---|---|---|
| *Reference Compound* | Roneparstat (SST0001) | ∼ 5 nM |
| *Reference Compound* | Suramin | ∼ 26 µM |
| **4** | SST0861AA1 | ++ |
| **7** | SST0863AA1 | + |
| **8** | SST0866AA1 | ++ |
| **9** | SST0880AA1 | + |
| **12** | SST0870AA1 | ++ |
| **19** | SST0564AA1 | + |
| **20** | SST0578AA1 | +++ |
| **33** | SST0587AA1 | + |
| **36** | SST0626AA1 | + |
| **40** | SST0665AA1 | + |
| **25** | SST0795AA1 | +++ |
| **26** | SST0797AA1 | ++ |
| **27** | SST0796AA1 | +++ |
| **28** | SST0862AA1 | + |
| **31** | SST0878AA1 | + |
| **38** | SST0872AA1 | +++ |

| | | |
|---|---|---|
| Legend: +: 1.00-20.00 µM ++: 0.50-0.99 µM +++: 0.05-0.49 µM | | |

From the results with a first screening on AGA*IA it is quite evident that the compounds of the present invention are endowed with high anti-heparanase activity. Some of them are active in the micromolar range (more potent than suramin), others are active in a submicromolar range. Some are very powerful, with an anti-heparanase activity less than 0.5 µM. Given the difference of M.W. with heparin-derivatives macromolecules currently under clinical investigation, it is very advantageous that these compounds are potent as Roneparstat, here used as reference compound. Roneparstat is a potent inhibitor of heparanase currently undergoing clinical trials.

Upon screening for inhibition of heparanase enzymatic activity, selected active compounds were further characterized *in vitro,* through a suitable cell proliferation assay (SRB Cell Cytotoxicity Assay) for their putative cytotoxic activity against three tumor cell lines, and through specific cellular assays for their putative anti-metastatic activity. According to these data selected compounds were also tested for invasion and adhesive properties and evaluated in a real-time quantitative PCR (qPCR) assay.

### Example 11

### Cytotoxicity assay

### Materials and methods

SRB Cell Cytotoxicity Assay is an assay based upon the quantitative staining of cellular proteins by sulforhodamine B (SRB). This assay can be used for high-throughput drug screening (Vichai & Kirkitara, Nature Protocol. 2006, 1, 112). Sulforhodamine B is an anionic aminoxanthene dye that forms an electrostatic complex with the basic amino acid residues of proteins under moderately acid conditions, which provides a sensitive linear response.

To assess a putative antiproliferative activity of the selected compounds, HT1080 (human fibrosarcoma), U87MG (human glioblastoma astrocytoma) and U2OS (human osteosarcoma) cell lines were treated for 72h, in triplicate, with increasing concentrations of heparanase inhibitors. Inhibition of cell proliferation was then measured by the classical colorimetric SRB Cell Cytotoxicity assay. Absorbance was measured by spectrophotometer at a wavelength of 510 nm and cell proliferation was determined as percent with respect to control cells (cells treated with the vehicle alone). EC₅₀ values were then determined by GraphPad Prism.

### Results

Results are summarized in the following Table 2.

**Table 2. Putative anti-proliferative activity of the selected compounds on three tumor cell lines.**

| | | | Concentration (µM) of test compounds at which the cell growth is inhibited by 50 %. | | |
|---|---|---|---|---|---|
| | | AGA*IA assay | HT1080 | U87MG | U2OS |
| *Reference compound* | Roneparstat (SST0001) | ∼5 nM | > 100 | > 100 | > 100 |
| *Reference compound* | Suramin | ∼ 26 µM | > 10 | > 10 | > 10 |
| 19 | SST0564AA1 | + | > 10 | > 10 | > 10 |
| 20 | SST0578AA1 | +++ | > 10 | > 10 | > 10 |
| 25 | SST0795AA1 | +++ | > 10 | > 10 | > 10 |
| 26 | SST0797AA1 | ++ | > 10 | > 10 | > 10 |
| 27 | SST0796AA1 | +++ | > 10 | > 10 | > 10 |
| 38 | SST0872AA1 | +++ | > 10 | > 10 | > 10 |

The selected compounds, all active as heparanase inhibitors in a submicromolar range, tested against three tumor cell lines up to 10 µM, showed a limited anti-proliferative activity, less than 50%.These data are very important because they show that compounds of the present invention, at concentrations in which they do not have a relevant interference with cell proliferation, are potent and selective heparanase inhibitors.

### Example 12

### Invasion and cell adhesion assays

### Materials and methods

### Invasion assay

Heparanase inhibitors were then evaluated for their activity on the invasive potential of HT1080 (human fibrosarcoma), U87MG (human glioblastoma astrocytoma) and U2OS (human osteosarcoma) human cell lines, using the Matrigel cell invasion assay which allows to assess the cell invasion ability of malignant and normal cells.

Invasion assay was performed using BioCoat cell culture inserts (BioCoat 8.0µm PET Membrane 24 Well Permeable Supports, Corning). Filters were coated with 100 µl of 300 µg/ml Matrigel dissolved in coating buffer (0.01 M Tris pH 8.0, 0.7% NaCl) (Becton-Dickinson Sciences). DMEM (Dulbecco's Modified Eagle's Medium) containing 5% FBS was added to the lower chambers. Cells were pre-treated with heparanase inhibitors for 24 h, detached and added to the upper chambers in media containing the inhibitors. Cells were allowed to invade for 24 h at 37°C in a CO₂ incubator and then unmigrated cells were removed from the upper chamber with a cotton swab. The invading cells were fixed, stained with 0.1% crystal violet for 10 min at room temperature, photographed and counted under inverted microscope.

Test compounds were analyzed at fixed concentration (2.5 µM) for several fields, in duplicates. Activity of test compounds was compared to the reference compound. Activity of most interesting compound was confirmed in a second cell invasion assay. Data were expressed as the percent invasion with respect to cell migration through uncoated insert membrane (see Table 3 below)

### Cell Adhesion Assays

Cell surface, non-integrin molecules such as heparan sulfate proteoglycans (HSPGs) may be involved in the regulation of early adhesive stages of cell-ECM interactions. A cell adhesion assay has been employed to determine if the newly designed heparanase inhibitors were able to interfere with adhesion properties of cancer cells.

HT1080 (human fibrosarcoma) and U87MG (human glioblastoma astrocytoma) cells were treated for 18-20 h with test compounds in complete medium supplemented with 10% FCS. Then cells were collected, washed in complete medium, and added to matrigel coated wells in triplicate, at previously identified optimal seeding density, and incubated at 37 °C in complete medium for times ranging from 15 minutes to 4h, according to the cell line. After incubation, the wells were washed three times with serum-free medium and the remaining firmly attached cells were stained with crystal-violet 0.1%. Optical density was measured at 560 nm by Victor 3 (Perkin-Elmer) plate analyzer. Each experiment was repeated three times.

### Results

Results are summarized in the following Table 3.

| Cpd | Code lab | Invasion Assay Inhibition | | | Adhesion Assay (%) | |
|---|---|---|---|---|---|---|
| | | HT1080 | U87MG | U2OS | HT1080 | U87MG |
| 20 | SST0578AA1 | + | ++ | ++ | 83.25 | 64.90 |
| 25 | SST0795AA1 | ++ | ++ | + | 81.60 | 12.30 |
| 27 | SST0796A1 | + | ++ | + | 90.94 | 61.10 |
| Reference compound | Suramin | + | - | - | 72.98 | 100 |

Table 3. Test compounds were analyzed at fixed concentration (2.5 µM) versus Reference compound (Suramin) at 10 µM. In Invasion assay, data were expressed as the percent invasion (range) with respect to cell migration through uncoated insert membrane. In Adhesion assay, data were expressed as the percent of adhesion with respect to the control (100%).
Legend
++: 50-90%
+ : < 50%
- : Not Active

The three selected compounds inhibited both invasion and adhesion. Invasion is known to be related to the heparanase activity unlike adhesion that is not directly related to the enzyme inhibition. Therefore, the effect on adhesion should be considered as an adjuvant advantageous effect of the tested compounds.

### Example 13

### Real-Time Quantitative PCR (qPCR) for measuring mRNA gene-expression of FGF1/2, VEGF, MMP-9, HSPE-1 in genes tumor cell lines

### Materials and methods

To evaluate the effect of heparanase inhibitors on the tumor transcription of genes encoding for proangiogenic factors, such as FGF1/2, VEGF, MMP-9, HSPE-1, the relative mRNA levels were analyzed by quantitative Real-Time Quantitative PCR. To perform the assay, HT1080 (human fibrosarcoma) cells were cultured in complete medium containing 10%FCS (fetal calf serum). Cells were plated in 24-well plates and treated at fixed concentration (i.e., highest non-toxic concentration) of test compounds. After 24 h, cells were detached, collected and total RNA extracted by RNeasy Mini Kit (QIAGEN). Following reverse transcription step, FGF1/2, VEGF, MMP-9, HSPE-1 mRNA levels, were quantified by qPCR using the advanced Universal SYBR Green Supermix (Bio Rad) and ABI PRISM 7900HT Thermal Cycler System (Perkin Elmer), according to the manufacturer's instructions. Appropriate no-RT and non-template controls were included in each 96-well PCR reaction, and dissociation analysis was performed at the end of each run to confirm the specificity of the reaction. Relative levels of RNA were calculated using the ddCt method. Standard curve and sample data were run in duplicate.

In the Table 5 a mean value is reported.

### Results

Results are summarized in the following Table 4.

**Table 4. Real-Time q-PCR of pro-angiogenic genes**

| | | Gene Expression (% mRNA vs. control) | | | | |
|---|---|---|---|---|---|---|
| | Conc | FGF-1 | FGF-2 | VEGF | MMP-9 | HPSE-1 |
| Control | | ∼ 100 | ∼ 100 | ∼ 100 | ∼ 100 | ∼ 100 |
| SST0578AA1 | 2.5 uM | ∼ 85 | ∼ 88 | ∼ 72 | ∼ 70 | ∼ 100 |
| SST0795AA1 | 10.0 uM | ∼ 52 | ∼ 68 | ∼ 63 | ∼ 65 | ∼ 62 |
| SST0001 | 10.0 uM | ∼ 70 | ∼ 77 | ∼ 43 | ∼ 42 | ∼ 70 |

Data from qPCR assay, with two selected active compounds versus SST0001 (reference compound) on HT1080 (human fibrosarcoma) cells, highlighted an inhibitory effect against the tumor transcription genes encoding for all proangiogenic factors tested.

From the comparison of the results of the two compounds we studied at different concentrations, it is clear that the inhibitory effect increases with the concentration. Less evident for SST0578 tested at 2.5 µM, more evident for SST0795 tested at 10 µM, as well as the reference product SST0001 (Roneparstat).

## Claims

1. A compound having the following formula (I) 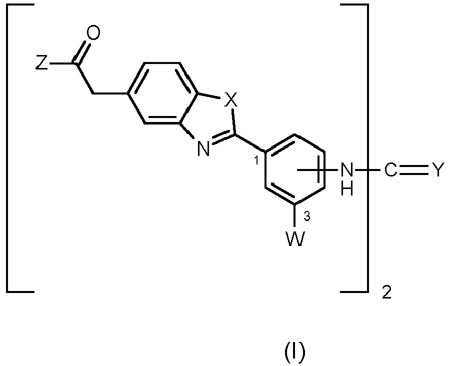
wherein Y is selected from the group consisting of O, S and NH,
X is selected from the group consisting of O and NH,
W is selected from the group consisting of H and F,
Z is selected from the group consisting of OH, NH-CHR-COOH, wherein R is the residue of an amino acid, and NHCHR₂R₃, wherein R₂ is selected from H and CH₂CH(CH₃)₂ and R₃ is selected from H and B(OH)₂,
the enantiomers, diastereoisomers, and mixtures thereof,
or a pharmaceutically acceptable salt, hydrate or solvate thereof.

2. The compound according to claim 1 wherein W is fluorine (F).

3. The compound according to claim 1 or 2 wherein Z is selected from OH and NH-CHR-COOH wherein R is the residue of an amino acid, said amino acid preferably selected from glycine, alanine and phenylalanine.

4. The compound according to anyone of the preceding claims wherein the group NHC=Y is in position 4 of the phenyl ring.

5. The compound according to anyone of the preceding claims, wherein W is F, Y is O, and X is O.

6. The compound according to claim 5 wherein Z is selected from OH and NH-CHR-COOH, wherein R is a residue of an amino acid.

7. The compound according to anyone of claims 1-4, wherein W is F, Y is O and X is NH.

8. The compound according to claim 7 wherein Z is selected from OH and NH-CHR-COOH, wherein R is a residue of an amino acid, said amino acid being preferably phenylalanine.

9. The compound according to anyone of claims 1-4 wherein W is F, Y is S and X is O.

10. The compound according to claim 9 wherein Z is NH-CHR-COOH, wherein R is a residue of an amino acid, said amino acid being preferably glycine.

11. The compound according to anyone of claims 1-4 wherein W is F, Y is S and X is NH.

12. The compound according to anyone of the preceding claims which is one of the followings:
2,2'-(((Carbonylbis(azanediyl))bis(3-fluoro-4,1-phenylene))bis(benzo[d]oxazole-2,5-diyl))diacetic acid,
2,2'-((2,2'-(((Carbonylbis(azanediyl))bis(3-fluoro-4,1-phenylene))bis(benzo[d]oxazole-2,5-diyl))bis(acetyl))bis(azanediyl))diacetic acid,
(2S,2'S)-2,2'-((2,2'-(((Carbonylbis(azanediyl))bis(3-fluoro-4,1-phenylene))bis(benzo[d]oxazole-2,5-diyl))bis(acetyl))bis(azanediyl))dipropionic acid, (2S,2'S)-2,2'-((2,2'-(((Carbonylbis(azanediyl))bis(3-fluoro-4,1-phenylene))bis(benzo[d]oxazole-2,5-diyl))bis(acetyl))bis(azanediyl))bis(3-phenylpropanoic acid),
2,2'-(((Carbonylbis(azanediyl))bis(3-fluoro-4,1-phenylene))bis(1H-benzo[d]imidazole-2,5-diyl))diacetic acid,
(2S,2'S)-2,2'-((2,2'-(((Carbonylbis(azanediyl))bis(3-fluoro-4,1-phenylene))bis(1H-benzo[d]imidazole-2,5-diyl))bis(acetyl))bis(azanediyl))bis(3-phenylpropanoic acid), 2,2'-(((Thiocarbonylbis(azanediyl))bis(3-fluoro-4,1-phenylene))bis(1H-benzo[d]imidazole-2,5-diyl))diacetic acid, and
2,2'-((2,2'-(((Thiocarbonylbis(azanediyl))bis(3-fluoro-4,1-phenylene))bis(benzo[d]oxazole-2,5-diyl))bis(acetyl))bis(azanediyl))diacetic acid.

13. The compound according to anyone of claims 1-11 which is one of the followings:
2,2'-((2,2'-(((carbonylbis(azanediyl))bis(3-fluoro-4,1-phenylene))bis(1*H-*benzo[*d*]imidazole-2,5-diyl))bis(acetyl))bis(azanediyl))diacetic acid,
(((2,2'-(((Carbonylbis(azanediyl))bis(3-fluoro-4,1-phenylene))bis(1H-benzo[d]imidazole-2,5-diyl))bis(acetyl))bis(azanediyl))bis(methylene))diboronic acid, 2,2'-(((Carbonylbis(azanediyl))bis(3-fluoro-4,1-phenylene))bis(benzo[d]oxazole-2,5-diyl))bis(N-isopentylacetamide),
(1R,1'R)-(((2,2'-(((Carbonylbis(azanediyl))bis(3-fluoro-4,1-phenylene))bis(benzo[d]oxazole-2,5-diyl))bis(acetyl))bis(azanediyl))bis(3-methylbutane-1,1-diyl))diboronic acid,
2,2'-(((Carbonylbis(azanediyl))bis(4,1-phenylene))bis(benzo[d]oxazole-2,5-diyl))diacetic acid,
2,2'-(((thiocarbonylbis(azanediyl))bis(4,1-phenylene))bis(benzo[d]oxazole-2,5-diyl))diacetic acid,
2,2'-(((Thiocarbonylbis(azanediyl))bis(3-fluoro-4,1-phenylene))bis(benzo[d]oxazole-2,5-diyl))diacetic acid and
2,2'-((((Iminomethylene)bis(azanediyl))bis(4,1-phenylene))bis(benzo[d]oxazole-2,5-diyl))diacetic acid.

14. The compound according to anyone of claims 1-13 for use as a medicament.

15. The compound according to anyone of claims 1-13 for use in the treatment of a disease which can be improved or prevented by an anti-heparanase activity.

16. The compound for the use according to claim 15 wherein said disease is selected from the group consisting of tumors, primary tumours, metastases, diabetic retinopathies, psoriasis, retrolenticular fibroplasia, restenosis after angioplasty, coronary by-pass, inflammation, sepsis, arthritis, autoimmune diseases, allograft rejection, cardiovascular diseases, fibro-proliferative disease, diseases elicited by abnormal platelet aggregation, diseases elicited by smooth muscle proliferation, Goodpasture syndrome, acute and chronic kidney diseases, glomerulonephritis, membranous nephropathy, diabetic nephropathy, neonatal pulmonary hypertension, asthma, congestive heart failure, adult pulmonary hypertension, renal vascular hypertension, proliferative retinopathies, experimental autoimmune encephalomyelitis, multiple sclerosis, insulin dependent diabetes, inflammatory bowel disease, ulcerative colitis, Crohn's disease, chronic inflammation, bone osteolysis, thrombosis, atherosclerosis, amyloidosis, hereditary multiple exostosis, bladder pain, vulvodynia, acute and chronic pancreatitis, empyema, diabetes, liver fibrosis, kidney fibrosis, peritoneal fibrosis, in particular peritoneal fibrosis glucose induced following peritoneal dialysis, and aging.

17. A pharmaceutical composition containing as active ingredient a compound according to anyone of claims 1-13 and at least one pharmaceutically acceptable vehicle and/or excipient.

18. The pharmaceutical composition according to claim 17 wherein said composition further comprises one or more further active ingredients.

19. The pharmaceutical composition according to claim 18 wherein said further active ingredient is a chemotherapeutic agent.

20. The pharmaceutical composition according to claim 19 wherein said chemotherapeutic agent is selected from the group consisting of cytotoxic agents, proteasome inhibitors, immunomodulatory drugs, topoisomerase inhibitors, kinase inhibitors and monoclonal antibodies, in particular directed towards highly expressed tumor associated antigens.

21. The pharmaceutical composition according to claim 20 wherein said chemotherapeutic agent is a cytotoxic agent selected from the group consisting of melphalan, irinotecan, rituximab and lapatinib; or is a proteasome inhibitor selected from the group consisting of bortezomib, carfilzomib and ixazomib; or is an immunomodulatory agent selected from the group consisting of thalidomide, lenalidomide and pomalidomide.
